# EUROPEAN PATENT SPECIFICATION

(11) **EP 3 257 488 B1**
(45) Date of publication and mention of the grant of the patent: **01.05.2019**
(21) Application number: 15882002.7
(22) Date of filing: 18.02.2015
(51) Int. Cl.: A61F 13/15, A61F 13/49

(54) **METHOD AND DEVICE FOR MANUFACTURING SHEET MEMBER FOR ABSORPTIVE ARTICLES**
VERFAHREN UND VORRICHTUNG ZUR HERSTELLUNG EINES BAHNENFÖRMIGEN ELEMENTS FÜR SAUGFÄHIGE ARTIKEL
PROCÉDÉ ET DISPOSITIF DE FABRICATION D'UN ÉLÉMENT FEUILLE POUR ARTICLE ABSORBANT

(30) Priority: 12.02.2015 JP 2015025020
(43) Date of publication of application: 20.12.2017
(73) Proprietor: Unicharm Corporation, Shikokuchuo-shi, Ehime 799-0111 (JP)
(72) Inventor: AKANO, Yukihisa, Kanonji-shi Kagawa 769-1602 (JP); MATSUO, Soya, Kanonji-shi Kagawa 769-1602 (JP)
(74) Representative: Staeger & Sperling Partnerschaftsgesellschaft mbB
(86) International application number: PCT/JP2015/054363
(87) International publication number: WO 2016/129128

(56) References cited:
- EP-A1- 2 415 430
- JP-A- 2004 254 862
- JP-A- 2007 260 414
- JP-A- 2010 240 112
- JP-A- 2012 115 358
- JP-A- 2013 138 795
- JP-A- 2014 511 753
- US-A1- 2005 230 037
- US-A1- 2013 255 862

## Description

### [Technical Field]

The present invention relates to a method and an apparatus for manufacturing sheet-like members associated with absorbent articles such as disposable diapers.

### [Background Art]

Sheet-like members, which each include a high stretchable region and a low stretchable region having lower elasticity than the high stretchable region in a predetermined direction, with the high stretchable region and the low stretchable region arranged in the predetermined direction, are conventionally used for manufacturing disposable diapers known as absorbent articles that absorb excreted fluid such as urine.

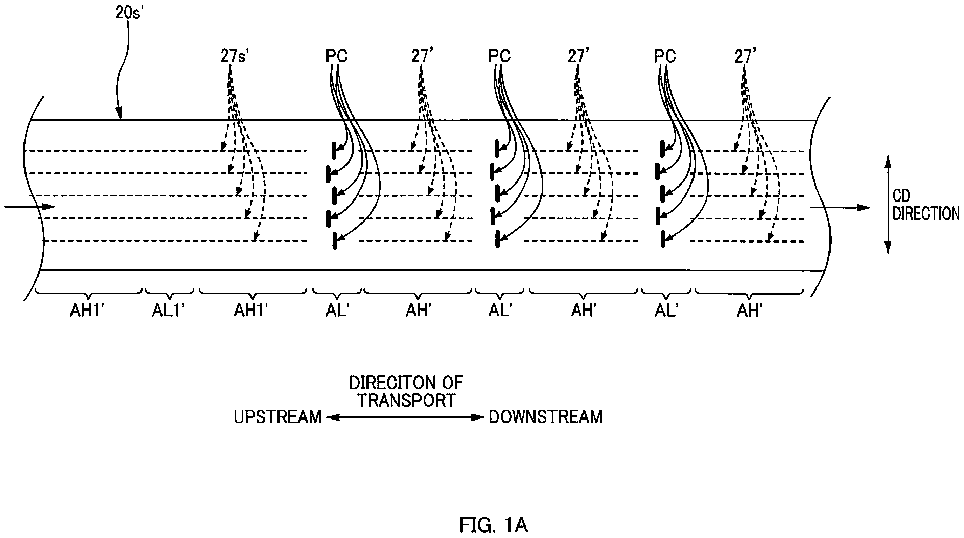

Such a sheet-like member 20s' is formed by the following manner. FIG. 1A is a diagram illustrating its producing process and a schematic plan view of the sheet-like member 20s'.

Firstly, the continuous sheet 20s' that is continuous in a predetermined direction is transported in the predetermined direction serving as the direction of transport. Further, a plurality of elastic members 27s' such as elastic strings that are in extended states in the above-mentioned direction of transport and that are sent in the direction of transport are supplied to the continuous sheet 20s' in a state with the plurality of elastic members 27s' arranged in the CD direction, intersecting the direction of transport. Then, the plurality of elastic members 27s' are fixed, to the continuous sheet 20s' with hot-melt adhesives etc. at a region AH1' that corresponds to a high stretchable region AH', and the plurality of elastic members 27s' are not fixed to the continuous sheet 20s' at a region AL1' that corresponds to a low stretchable region AL'. Thereafter, each of the plurality of elastic members 27s', 27s' ... is cut at its own one location PC in the direction of transport within the region AL1' that corresponds to the low stretchable region AL'. The elastic members 27' which has been cut are made to contract toward regions AH1' each of which corresponds to the high stretchable regions AH'. And, hereby, the high stretchable regions AH' and the low stretchable regions AL' are formed and arranged in the direction of transport onto the continuous sheet 20s'.

### [Citation List]

### [Patent Literature]

[PTL 1]
Japanese Unexamined Patent Application Publication (Translation of PCT Application) No. 2014-511753

### [Summary of Invention]

### [Technical Problem]

The abovementioned elastic members 27s', 27s' ... are cut with a cutter device (not-shown), for example. The cutter device includes a cutter roll and an anvil roll that rotates with their respective outer circumferential surfaces opposing each other. And, when the continuous sheet 20s' on which the elastic members 27s', 27s' ... shown in FIG. 1A are fixed passes through between the rotating cutter and anvil rolls, the elastic members 27s', 27s' ... are cut by pressing the elastic members 27s', 27s' ... and the continuous sheet 20s' in the thickness direction with the anvil roll and the cutter blades C', C' ... on the outer circumferential surface of the cutter roll.

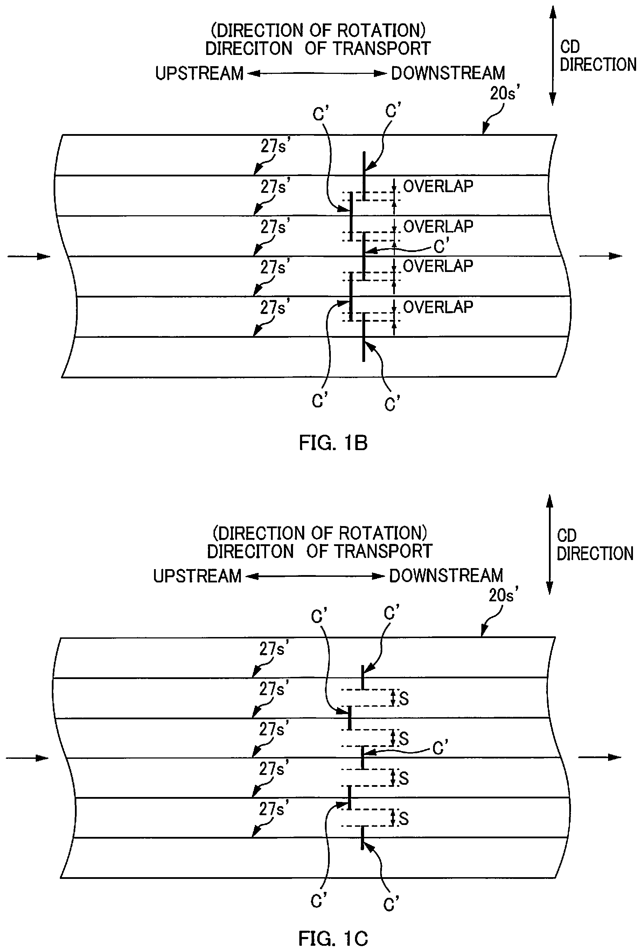

FIGS. 1B and 1C show the first reference example and the second reference example of a pattern in which the cutter blades C', C' ... are arranged. Both of the figures are schematic views illustrating the outer circumferential surface of the cutter roll developed on a plane. These figures also virtually indicate the elastic members 27s' with the continuous sheet 20s'.

Basically in both of the reference examples, the cutter blades C' are each arranged corresponding to the respective elastic members 27s'. Hereby, each elastic member 27s' is cut at its own one location PC (FIG. 1A) in the direction of transport as described above. The position where each cutter blade C' cuts the elastic member 27s' corresponds to substantially the center position of the cutter blade C' in the CD direction.

In the first reference example of FIG. 1B, cutter blades C' and C' adjacent in the CD direction are continuously arranged in the CD direction. In the example of FIG. 1B, each pair of the adjacent cutter blades C' and C' overlap in the CD direction. Accordingly, the elastic members 27s' are kept from failing to be cut or be cut cleanly even when the elastic members 27s' shifts in the CD direction due to meandering or contraction of the continuous sheet 20s' or the like.

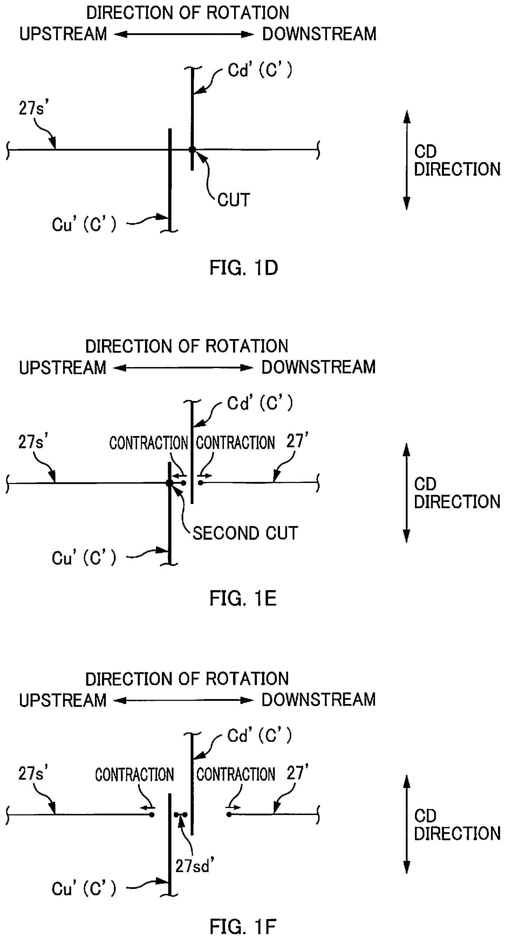

However, in this case, as shown in the schematic magnified view of FIG. 1D, the cutter blades Cu' and Cd' that are arranged continuously in the CD direction side by side in the direction of rotation of the cutter roll. And, as shown in FIGS 1D and 1E, when the elastic member 27s' that has cut by the downstream cutter blade Cd' is contracting upstream in the direction of transport (the direction of rotation), there is a possibility that an unintentional second cut is made by the upstream cutter blade Cu'. In such a case, a shredded piece 27sd' of the elastic member 27s' will be produced, as shown in FIG. 1F. And, a diaper made of a continuous sheet 20s' which includes such a shredded piece 27sd' is possibly discarded as a defective product that includes a foreign matter thereby possibly causing product yield loss.

On the other hand, in the second reference example of FIG. 1C, each pair of cutter blades C' and C' adjacent in the CD direction are arranged in such a manner that the cutter blades do not continue in the CD direction. That is, between these cutter blades C' and C', there is a space S in the CD direction. This makes it possible to avoid promptly the foregoing second cut, and can prevent producing such a shredded piece 27sd'. But, the elastic members 27s' will fail to be cut or be cut cleanly when the elastic members 27' were to move to the position of the space S by the shifting of the elastic members 27s' in the CD direction.

That is, both arrangement patterns in the first and second reference examples have their own advantages and disadvantages.

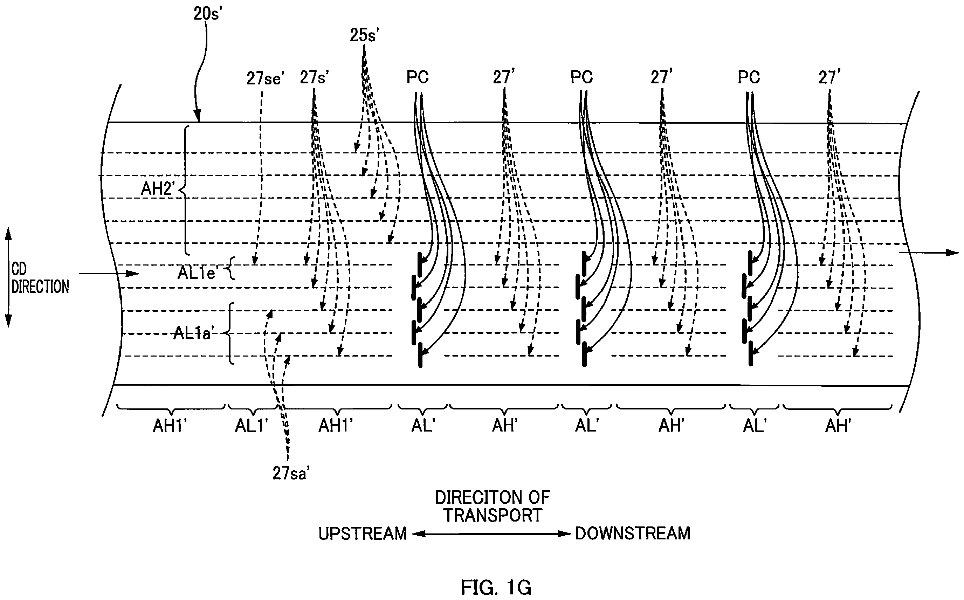

Depending on a type of diaper, on the continuous sheet 20s', there may be a position at which a reliable cutting of the elastic members 27s' has a priority, and a position at which prevention of producing the shredded pieces 27sd' has a priority. FIG. 1G is a schematic diagram illustrating an example thereof. That is, continuous sheet 20s' shown in FIG. 1G further includes a second region AH2', which is adjacent in the CD direction to the regions AL1' (corresponding to the low stretchable region AL') and the regions AH1' (corresponding to the high stretchable region AH'). To the second region AH2', fixed are a plurality of second elastic members 25s', 25s' ... extending in the direction of transport and being continuous in the direction of transport; thus, the second region AH2' is a second high stretchable region AH2'. In this case, in an end part AL1e' of the region AL1' in the CD direction that is on the side closer to the second high stretchable region AH2', even if an elastic member 27se' fails to be cut or be cut cleanly, it can be considered that damage to low stretchability of the low stretchable region AL' is small. That is, since the position of such cutting failure is near the second high stretchable region AH2', the one elastic member 27se' that has failed to be cut or be cut cleanly can be considered to be additional one no-cut elastic member 25' in the second high stretchable region AH2'. Consequently, it is possible to substantially ignore the cutting failure. On the other hand, as shown in FIG. 1G, at a position AL1a' apart from the second high stretchable region AH2' in the CD direction, if an elastic member 27sa' associated with the low stretchable region AL' has failed to be cut or be cut cleanly, a stretchability that is not negligible is applied to the low stretchable region AL'. Consequently, low stretchability of the low stretchable region AL' is significantly damaged.

That is, at a position like the end part AL1e', prevention of producing the shredded pieces 27sd' may have a priority; and on the other hand, at a position like the position ALla', a reliable cutting of the elastic members 27s' may have a priority.

Prevention of producing the shredded pieces 27sd' can be achieved by an arrangement pattern in which cutter blades C' and C' adjacent in the CD direction are arranged discontinuously in the CD direction, like the arrangement pattern shown in the second reference example of FIG. 1C. On the other hand, preventing of cutting failure can be achieved by an arrangement pattern in which cutter blades C' and C' adjacent in the CD direction are arranged continuously in the CD direction like the arrangement pattern shown in the first reference example of FIG. 1B.

The present invention has been made in view of conventional problems such as that described above and an objective thereof is to enable to prepare on the continuous sheet a cutting-failure preventing position which can prevent elastic members from failing to be cut or be cut cleanly and a shredded-piece preventing position which can prevent producing shredded pieces of the elastic members.

### [Solution to Problem]

An aspect of the invention to achieve the above advantage is a method of manufacturing a sheet-like member associated with an absorbent article,
the sheet-like member having a high stretchable region and a low stretchable region that has a lower stretchability than the high stretchable region in a predetermined direction,
the high stretchable region and the low stretchable region being arranged in the predetermined direction,
the method including:
   transporting a continuous sheet along a direction of transport that is the predetermined direction,
      the continuous sheet including a plurality of elastic members that are continuous along the predetermined direction in an extending state in the predetermined direction,
      the plurality of elastic members being in a state lined in a CD direction that intersects the predetermined direction,
      the plurality of elastic members being fixed at least to a region corresponding to the high stretchable region; and
   forming the high stretchable region and the low stretchable region
      so as to be arranged in the direction of transport in the continuous sheet
      by cutting each of the plurality of elastic members at a position in the direction of transport in a region corresponding to the low stretchable region and
      by allowing the plurality of elastic members to contract toward the region corresponding to the high stretchable region,
   wherein
   when the continuous sheet to which the plurality of elastic members are fixed passes along the direction of transport between a cutter rotating body and an anvil rotating body that rotate with respective outer circumferential surfaces facing each other,
      the plurality of elastic members are cut by pressing the continuous sheet by the anvil rotating body and a plurality of cutter blades on the outer circumferential surface of the cutter rotating body,
   the continuous sheet includes a first position and a second position,
      the first position being a position at which cutter blades adjacent in the CD direction are arranged continuously in the CD direction,
      the second position being a position at which cutter blades adjacent in the CD direction are arranged discontinuously in the CD direction,
   a size of a space in the CD direction between the cutter blades arranged discontinuously in the second position is smaller than a minimum of an inter-center distance in the CD direction between adjacent elastic members of the plurality of elastic members,
      the adjacent elastic members being adjacent in the CD dire ction.

Further, an apparatus of manufacturing a sheet-like member associated with an absorbent article,
the sheet-like member having a high stretchable region and a low stretchable region that has a lower stretchability than the high stretchable region in a predetermined direction,
the high stretchable region and the low stretchable region being arranged in the predetermined direction,
the apparatus including:
   a transporting device that transports a continuous sheet along a direction of transport that is the predetermined direction,
      the continuous sheet including a plurality of elastic members that are continuous along the predetermined direction in an extending state in the predetermined direction,
      the plurality of elastic members being in a state lined in a CD direction that intersects the predetermined direction,
      the plurality of elastic members being fixed at least to a region corresponding to the high stretchable region; and
   a cutter device that forms the high stretchable region and the low stretchable region
      so as to be arranged in the direction of transport in the continuous sheet
      by cutting each of the plurality of elastic members at a position in the direction of transport in a region corresponding to the low stretchable region and
      by allowing the plurality of elastic members to contract toward the region corresponding to the high stretchable region,
      the cutter device including a cutter rotating body and an anvil rotating body,
   wherein
   when the continuous sheet to which the plurality of elastic members are fixed passes along the direction of transport between the cutter rotating body and the anvil rotating body that rotate with respective outer circumferential surfaces facing each other,
      the plurality of elastic members are cut by pressing the continuous sheet by the anvil rotating body and a plurality of cutter blades on the outer circumferential surface of the cutter rotating body,
   the continuous sheet includes a first position and a second position,
      the first position being a position at which cutter blades adjacent in the CD direction are arranged continuously in the CD direction,
      the second position being a position at which cutter blades adjacent in the CD direction are arranged discontinuously in the CD direction,
   a size of a space in the CD direction between the cutter blades arranged discontinuously in the second position is smaller than a minimum of an inter-center distance in the CD direction between centers of a pair of elastic members of the plurality of elastic members,
      the pair of elastic members being adjacent in the CD direction.

Features of the present invention other than the above will become clear by reading the description of the present specification with reference to the accompanying drawings.

### [Advantageous Effects of Invention]

According to the present invention, it is possible to prepare on the continuous sheet a cutting-failure preventing position which can prevent elastic members from failing to be cut or be cut cleanly and a shredded-piece preventing position which can prevent producing shredded pieces of the elastic members.

### [Brief Description of Drawings]

FIG. 1A is a schematic plan view illustrating a producing process of a sheet-like member 20s' having a high stretchable region AH' and a low stretchable region AL'.
FIG. 1B is a schematic view illustrating of a first reference example of a pattern in which cutter blades C', C' ... are arranged.
FIG. 1C is a schematic view illustrating of a second reference example of a pattern in which the cutter blades C', C' ... are arranged.
FIG. 1D is a diagram illustrating a problem of a shredded piece of an elastic member 27s' that may be created with the pattern in which the cutter blades C', C' ... are arranged in the first reference example.
FIG. 1E is an explanatory diagram similar to FIG. 1D.
FIG. IF is an explanatory diagram similar to FIG. 1D.
FIG. 1G is a schematic diagram illustrating the presence of the following positions on a continuous sheet 20s': a position at which a reliable cutting of elastic members 27s' has a priority; and a position at which prevention of producing shredded pieces 27sd' has a priority.
FIG. 2 is a schematic perspective view of a three-piece type disposable diaper 1 as an example of an absorbent article associated with the present embodiment.
FIG. 3 is a schematic plan view of the unfolded diaper 1 seen from a skin side.
FIG. 4 is a schematic plan view of the diaper 1 seen from a non-skin side.
FIG. 5A is a cross-sectional view taken along line A-A of FIG. 3, and FIG. 5B is a cross-sectional view taken along line B-B of FIG. 3.
FIG. 6 is a cross-sectional view taken along line VI-VI of FIG. 3.
FIG. 7 is a schematic plan view of a front band member 20a seen from the non-skin side.
FIG. 8 is a schematic plan view of the front band member 20a seen from the non-skin side before elastic string continuous bodies 27s are cut.
FIG. 9A is a schematic plan view of a sheet-like member 20as immediately before being transported to a manufacturing apparatus 30 of the present embodiment, FIG. 9B is a schematic plan view of the sheet-like member 20as in which the stretchable regions AH, AH and the non-stretchable region AL are formed by the manufacturing apparatus 30.
FIG. 10A is a schematic side view of the manufacturing apparatus 30, and FIG. 10B is a view along arrows B-B in FIG. 10A.
FIG. 11 is an explanatory diagram showing a state of arrangement of a plurality of cutter blades C, C ... provided in a cutter block 54 of a cutter roll 51d and showing a state in which the outer circumferential surface 51da of the roll 51d is opened on a plane.
FIG. 12 is a diagram illustrating a first modified example of a shredded-piece preventing position.
FIG. 13 is a diagram illustrating a second modified example of the shredded-piece preventing position.
FIG. 14 is a diagram illustrating a third modified example of the shredded-piece preventing position.
FIG. 15 is a schematic plan view of a first modified example of an arrangement pattern of the cutter blades C, C ....
FIG. 16 is a schematic plan view of a second modified example of an arrangement pattern of the cutter blades C, C ....

### [Description of Embodiments]

At least the following matters will become clear with the description of this specification and the attached drawings.

A method of manufacturing a sheet-like member associated with an absorbent article,
the sheet-like member having a high stretchable region and a low stretchable region that has a lower stretchability than the high stretchable region in a predetermined direction,
the high stretchable region and the low stretchable region being arranged in the predetermined direction,
the method including:
   transporting a continuous sheet along a direction of transport that is the predetermined direction,
      the continuous sheet including a plurality of elastic members that are continuous along the predetermined direction in an extending state in the predetermined direction,
      the plurality of elastic members being in a state lined in a CD direction that intersects the predetermined direction,
      the plurality of elastic members being fixed at least to a region corresponding to the high stretchable region; and
   forming the high stretchable region and the low stretchable region
      so as to be arranged in the direction of transport in the continuous sheet
      by cutting each of the plurality of elastic members at a position in the direction of transport in a region corresponding to the low stretchable region and
      by allowing the plurality of elastic members to contract toward the region corresponding to the high stretchable region,
   wherein
   when the continuous sheet to which the plurality of elastic members are fixed passes along the direction of transport between a cutter rotating body and an anvil rotating body that rotate with respective outer circumferential surfaces facing each other,
      the plurality of elastic members are cut by pressing the continuous sheet by the anvil rotating body and a plurality of cutter blades on the outer circumferential surface of the cutter rotating body,
   the continuous sheet includes a first position and a second position,
      the first position being a position at which cutter blades adjacent in the CD direction are arranged continuously in the CD direction,
      the second position being a position at which cutter blades adjacent in the CD direction are arranged discontinuously in the CD direction,
   a size of a space in the CD direction between the cutter blades arranged discontinuously in the second position is smaller than a minimum of an inter-center distance in the CD direction between adjacent elastic members of the plurality of elastic members,
      the adjacent elastic members being adjacent in the CD direction.

With such a method of manufacturing a sheet-like member associated with an absorbent article, at the first position, the cutter blades adjacent in the CD direction are arranged continuously in the CD direction. Even when a near-located elastic member shifts in the CD direction, it is possible to prevent the elastic members from failing to be cut or be cut cleanly. On the other hand, at the second position, the cutter blades adjacent in the CD direction are arranged in such a manner that the cutter blades do not continue in the CD direction. Accordingly, after the downstream cutter blade in the direction of rotation cuts the elastic member, it is possible to prevent the upstream cutter blade from making a second cut to the same elastic member being contracting upstream in the direction of rotation. This makes it possible to prevent producing shredded pieces of the elastic member. This makes it possible to prepare on the continuous sheet a cutting-failure preventing position which can prevent the elastic member from failing to be cut or be cut cleanly and a shredded-piece preventing position which can prevent producing shredded pieces of the elastic member.

In such a method of manufacturing a sheet-like member associated with an absorbent article, it is desirable
that the continuous sheet includes a second region that is adjacent in the CD direction to a region corresponding to the low stretchable region and that is adjacent in the CD direction to a region corresponding to the high stretchable region,
that a plurality of second elastic members are fixed to the second region,
   the plurality of second elastic members being continuous along the predetermined direction in an extending state in the predetermined direction,
   the plurality of second elastic members being in a state lined in the CD direction that intersects the predetermined direction,
   the second region being defined as a second high stretchable region, and
that the second position is set in an end part of the region corresponding to the low stretchable region,
   the end part being located on a side closer to the second high stretchable region in the CD direction.

With such a method of manufacturing a sheet-like member associated with an absorbent article, in the region corresponding to the low stretchable region, the second position is set in the end part in the CD direction located on a side closer to the second high stretchable region. Thus, in the end part, it is possible to prevent producing shredded pieces. Even if, an elastic member fails to be cut or be cut cleanly in the end part, it can be considered that the degree of damage to low stretchability of the low stretchable region is small. That is, the end part is near the second high stretchable region; therefore, even if the elastic member fails to be cut or be cut cleanly at a position of the end part, the elastic member which has failed to be cut or be cut cleanly can be considered as an additional elastic member in the second high stretchable region. Consequently, it is possible to substantially ignore the cutting failure.

In such a method of manufacturing a sheet-like member associated with an absorbent article, it is desirable
that the continuous sheet includes a second region that is adjacent in the CD direction to a region corresponding to the low stretchable region and that is adjacent in the CD direction to a region corresponding to the high stretchable region,
that a single-cut sheet is fixed to the region corresponding to the low stretchable region and is spaced from another single-cut sheet adjacent in the direction of transport, and
that the second position is set in an end part of the single-cut sheet,
   the end part being located on a side closer to the second region in the CD direction.

With such a method of manufacturing a sheet-like member associated with an absorbent article, the continuous sheet is likely to deform in the CD direction in the end part of the single-cut sheet located on a side closer to the second region in the CD direction; this deformation is due to difference of rigidity from a near position in which the single-cut sheet does not exist. Accordingly, an elastic member located in the end part is likely to shift in the CD direction. Consequently, there is a possibility that the elastic member moves to or near the boundary position of cutter blades adjacent in the CD direction. But, in this regard, the second position is set in the end part; therefore, the cutter blades are arranged discontinuously in the CD direction. More specifically speaking, a space in the CD direction is disposed between the cutter blades. Even when the elastic member shifts in the CD direction and moves to or near the boundary position of the cutter blades, it is possible to avoid promptly a second cut of the elastic member by the two cutter blades. This makes it possible to prevent producing shredded pieces of the elastic member.

In such a method of manufacturing a sheet-like member associated with an absorbent article, it is desirable
that the continuous sheet includes a second region that is adjacent in the CD direction to a region corresponding to the low stretchable region and that is adjacent in the CD direction to a region corresponding to the high stretchable region,
that adhesive is applied to the second region,
that a plurality of second elastic members are fixed to the second region with the adhesive,
   the plurality of second elastic members being continuous along the predetermined direction in an extending state in the predetermined direction,
   the plurality of second elastic members being in a state lined in the CD direction that intersects the predetermined direction,
that the second region is defined as a second high stretchable region,
that the plurality of elastic members are not fixed with adhesive to the region corresponding to the low stretchable region, and
that the second position is set in an end part of the region corresponding to the low stretchable region,
   the end part being located on a side closer to the second region in the CD direction.

With such a method of manufacturing a sheet-like member associated with an absorbent article, the continuous sheet is likely to deform in the CD direction in the end part of the region corresponding to the low stretchable region located on a side closer to the second region in the CD direction; this deformation is due to difference of rigidity from the second region, which is position near the end part and to which adhesive is applied. Accordingly, an elastic member located in the end part is likely to shift in the CD direction. Consequently, there is a possibility that the elastic member moves to or near the boundary position of cutter blades adjacent in the CD direction. But, in this regard, the second position is set in the end part; therefore, cutter blades for the end part are arranged discontinuously in the CD direction. More specifically speaking, a space in the CD direction is disposed between the cutter blades. Even when the elastic member shifts in the CD direction and moves to or near the boundary position of the cutter blades, it is possible to avoid promptly a second cut of the elastic member by the two cutter blades. This makes it possible to prevent producing shredded pieces of the elastic member.

In such a method of manufacturing a sheet-like member associated with an absorbent article, it is desirable that
a size of a space in the CD direction between the cutter blades that are arranged discontinuously at the second position is smaller than a thickness of an elastic member positioned nearest the second position among the plurality of elastic members.

With such a method of manufacturing a sheet-like member associated with an absorbent article, the size of the space in the CD direction is smaller than the thickness of the elastic member. This makes it possible for the cutter blades to cut the elastic member even if the elastic member moves to the space. Thus, at the second position, it is possible to effectively prevent cutting failure of the elastic members.

In such a method of manufacturing a sheet-like member associated with an absorbent article, it is desirable that
a size of a space in the CD direction between the cutter blades that are arranged discontinuously at the second position is equal to or greater than a thickness of an elastic member that is positioned nearest the second position among the plurality of elastic members

With such a method of manufacturing a sheet-like member associated with an absorbent article, the size of the space in the CD direction is equal to or greater than the thickness of the elastic member. This makes it possible to effectively avoid a second cut of the elastic member by these two cutter blades even if the elastic member moves to the space. Accordingly, it is possible to reliably prevent producing shredded pieces of the elastic member.

In such a method of manufacturing a sheet-like member associated with an absorbent article, it is desirable that
the cutter blades that are arranged continuously at the first position overlap in the CD direction.

With such a method of manufacturing a sheet-like member associated with an absorbent article, the cutter blades overlap in the CD direction. Thus, at the first position, it is possible to reliably prevent cutting failure of the elastic member.

In such a method of manufacturing a sheet-like member associated with an absorbent article, it is desirable that
the cutter blade is tilted with respect to a direction along an axis of rotation of the cutter rotating body.

With such a method of manufacturing a sheet-like member associated with an absorbent article, the cutter blade is tilted with respect to the direction along the axis of rotation of the cutter rotating body. Accordingly, it is possible to ensure a long cutting time for cutting the elastic member by the tilted amount of the cutter blade, in comparison with a case in which the cutter blade is parallel to the direction along the axis of rotation. This also effectively contributes to prevention cutting failure of elastic members.

In such a method of manufacturing a sheet-like member associated with an absorbent article, it is desirable that
among the plurality of cutter blades,
at least one another cutter blade is located in a direction of rotation of the cutter rotating body at a position between the cutter blades that are arranged continuously in the CD direction at the first position.

With such a method of manufacturing a sheet-like member associated with an absorbent article, the at least one other cutter blade is located in the direction of rotation at the position between the cutter blades that are arranged continuously in the CD direction at the first position, and the foregoing two cutter blades at the first position are moderately set apart in the direction of rotation. Accordingly, a moderate amount of time difference can be secured between these two cutter blades, one of which cuts prior to the other one and the other of which cuts afterward. This makes it possible to effectively prevent producing shredded pieces of the elastic member, which is caused by cooperation of the cutter blade which cuts prior to the other one and the cutter blade which cuts afterward. That is, after the downstream cutter blade cuts the elastic member, it is possible to prevent the upstream cutter blade from making a second cut to the elastic member being contracting upstream in the direction of rotation.

In such a method of manufacturing a sheet-like member associated with an absorbent article, it is desirable that am
ong the plurality of cutter blades,
at least one another cutter blade is located in a direction of rotation of the cutter rotating body at a position between the cutter blades that are arranged discontinuously in the CD direction at the second position.

With such a method of manufacturing a sheet-like member associated with an absorbent article, the at least one other cutter blade is located in the direction of rotation at the position between the cutter blades that are arranged discontinuously in the CD direction at the second position, and the foregoing two cutter blades at the second position are moderately set apart in the direction of rotation. Accordingly, a moderate amount of time difference can be secured between these two cutter blades, one of which cuts prior to the other one and the other of which cuts afterward. This makes it possible to effectively prevent producing shredded pieces of the elastic member, which is caused by cooperation of the cutter blade which cuts prior to the other one and the cutter blade which cuts afterward. That is, after the downstream cutter blade cuts the elastic member, it is possible to prevent the upstream cutter blade from making a second cut to the elastic member being contracting upstream in the direction of rotation.

In such a method of manufacturing a sheet-like member associated with an absorbent article, it is desirable
that after one cutter blade among the plurality of cutter blades has cut a certain elastic member and while the one cutter blade is still being pressed toward the anvil rotating body,
   another cutter blade among the plurality of cutter blades that subsequently cuts another elastic member is already being pressed toward the anvil rotating body, and
that at least one cutter blade is positioned in the CD direction between the one cutter blade and the other cutter blade.

With such a method of manufacturing a sheet-like member associated with an absorbent article, after the one cutter blade has cut a certain elastic member and while the one cutter blade is still being pressed toward the anvil rotating body, the other cutter blade that subsequently cuts another elastic member is already in a state being pressed toward the anvil rotating body. Thus, the reaction force of cutting that may act on the cutter rotating body accompanying the cutting can be received, after the cutting, not only by the one cutter blade but also by the other cutter blade. Accordingly, such a two-point supporting state makes it possible to reduce vibration of the cutter rotating body, which may be caused accompanying a fluctuation of the reaction force of the cutting after the elastic member is cut. This makes it possible to effectively restrain the vibration of the cutter rotating body. This also effectively contributes to prevention cutting failure of elastic members.

Further, at least one cutter blade is located in the CD direction between the one cutter blade and the other cutter blade. Accordingly, the cutter blades in the two-point supporting state can be disposed greatly apart from each other in the CD direction. That is, the span of the two-point supporting can be larger. This can effectively contribute to stabilization of the two-point supporting state. Consequently, the vibration of the cutter rotating body can be surely reduced.

In such a method of manufacturing a sheet-like member associated with an absorbent article, it is desirable that
a cutter blade that corresponds to the at least one cutter blade is present to all of cutter blades of the plurality of cutter blades other than a cutter blade that cuts the elastic member last.

With such a method of manufacturing a sheet-like member associated with an absorbent article, all of the cutter blades other than the cutter blade that cuts the elastic member last can also be put into the foregoing two-point supporting state comparatively with a cutter blade corresponding to the above-mentioned at least one cutter blade, when at least after the elastic member is cut. This makes it possible to effectively restrain the vibration of the cutter rotating body.

In such a method of manufacturing a sheet-like member associated with an absorbent article, it is desirable that
when the one cutter blade cuts the certain elastic member, the other cutter blade is already in a state being pressed toward the anvil rotating body.

With such a method of manufacturing a sheet-like member associated with an absorbent article, the other cutter blade is already in a state being pressed toward the anvil rotating body not only after the one cutter blade has cut the elastic member but also during the cutting. Thus, the reaction force of cutting that may act on the cutter rotating body accompanying the cutting can be received, when the cutting takes place, not only by the one cutter blade but also by the other cutter blade. Accordingly, such a two-point supporting state by the one cutter blade and the other cutter blade makes it possible to promptly reduce the vibration of the cutter rotating body, which may be caused accompanying a fluctuation of the reaction force of the cutting.

In such a method of manufacturing a sheet-like member associated with an absorbent article, it is desirable that
a number of the cutter blades that are simultaneously pressed toward the anvil rotating body among all of the plurality of cutter blades are set to two or less.

With such a method of manufacturing a sheet-like member associated with an absorbent article, the number of the cutter blades that are simultaneously pressed toward the anvil rotating body are set to two or less. This makes it possible to effectively prevent the pressing force acting on the cutter blades from decreasing due to distribution of the pressing force among a number of the cutter blades. Consequently, each of the elastic members can be surely cut.

Further, an apparatus of manufacturing a sheet-like member associated with an absorbent article,
the sheet-like member having a high stretchable region and a low stretchable region that has a lower stretchability than the high stretchable region in a predetermined direction,
the high stretchable region and the low stretchable region being arranged in the predetermined direction,
the apparatus including:
   a transporting device that transports a continuous sheet along a direction of transport that is the predetermined direction,
      the continuous sheet including a plurality of elastic members that are continuous along the predetermined direction in an extending state in the predetermined direction,
      the plurality of elastic members being in a state lined in a CD direction that intersects the predetermined direction,
      the plurality of elastic members being fixed at least to a region corresponding to the high stretchable region; and
   a cutter device that forms the high stretchable region and the low stretchable region
      so as to be arranged in the direction of transport in the continuous sheet
      by cutting each of the plurality of elastic members at a position in the direction of transport in a region corresponding to the low stretchable region and
      by allowing the plurality of elastic members to contract toward the region corresponding to the high stretchable region,
      the cutter device including a cutter rotating body and an anvil rotating body,
   wherein
   when the continuous sheet to which the plurality of elastic members are fixed passes along the direction of transport between the cutter rotating body and the anvil rotating body that rotate with respective outer circumferential surfaces facing each other,
      the plurality of elastic members are cut by pressing the continuous sheet by the anvil rotating body and a plurality of cutter blades on the outer circumferential surface of the cutter rotating body,
   the continuous sheet includes a first position and a second position,
      the first position being a position at which cutter blades adjacent in the CD direction are arranged continuously in the CD direction,
      the second position being a position at which cutter blades adjacent in the CD direction are arranged discontinuously in the CD direction,
   a size of a space in the CD direction between the cutter blades arranged discontinuously in the second position is smaller than a minimum of an inter-center distance in the CD direction between centers of a pair of elastic members of the plurality of elastic members,
      the pair of elastic members being adjacent in the CD direction.

With such an apparatus of manufacturing a sheet-like member associated with an absorbent article, the effect equivalent to those of the foregoing methods can be realized.

### Present Embodiment

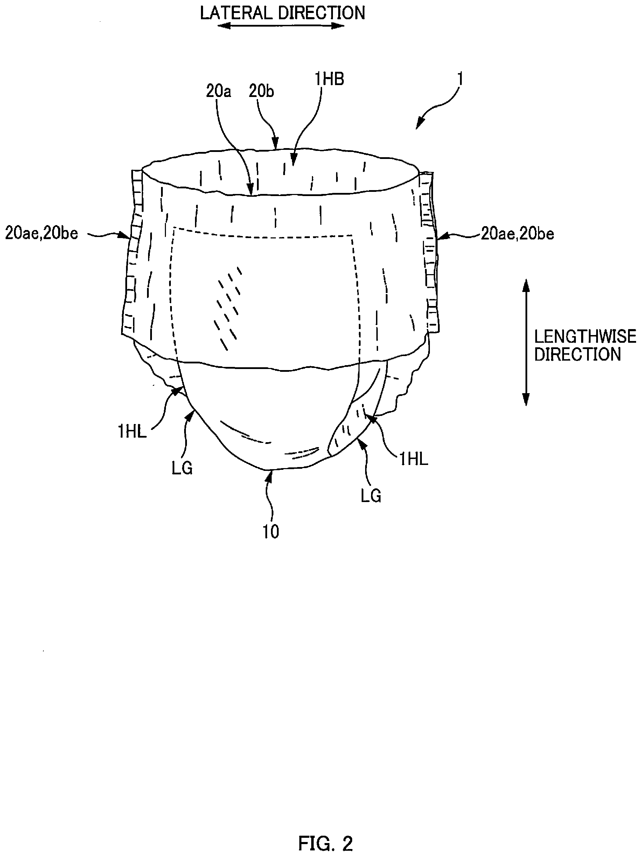

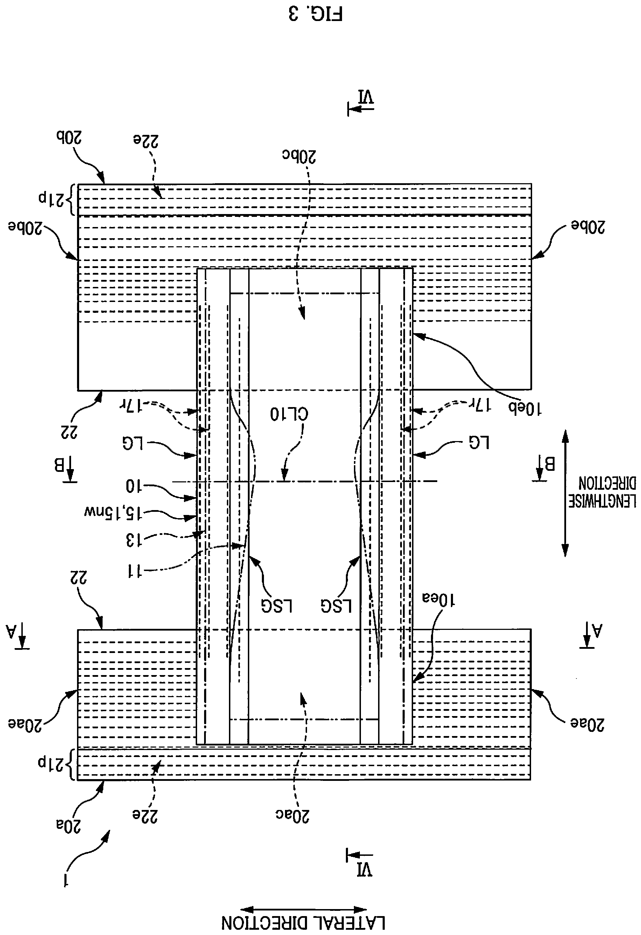

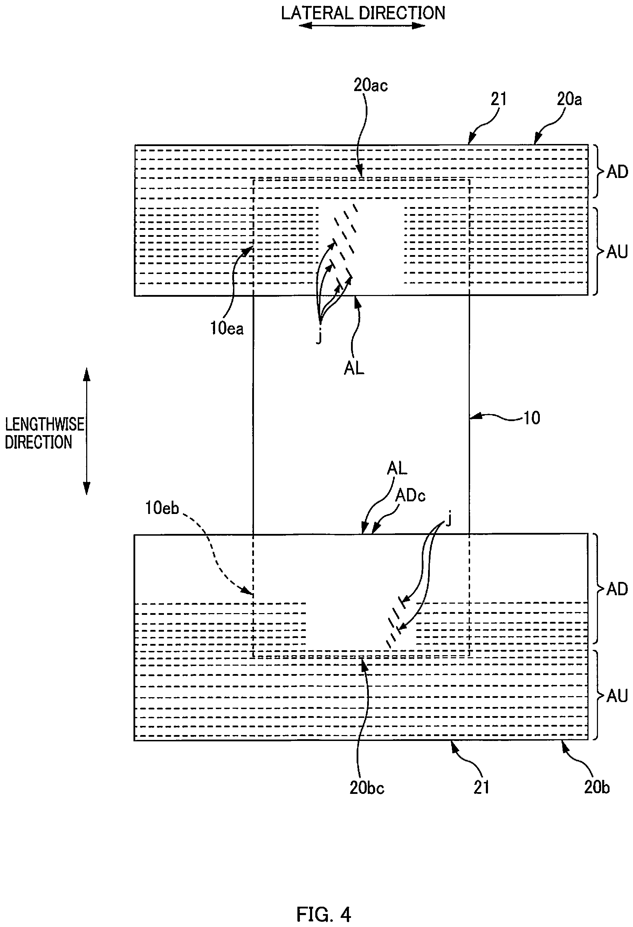

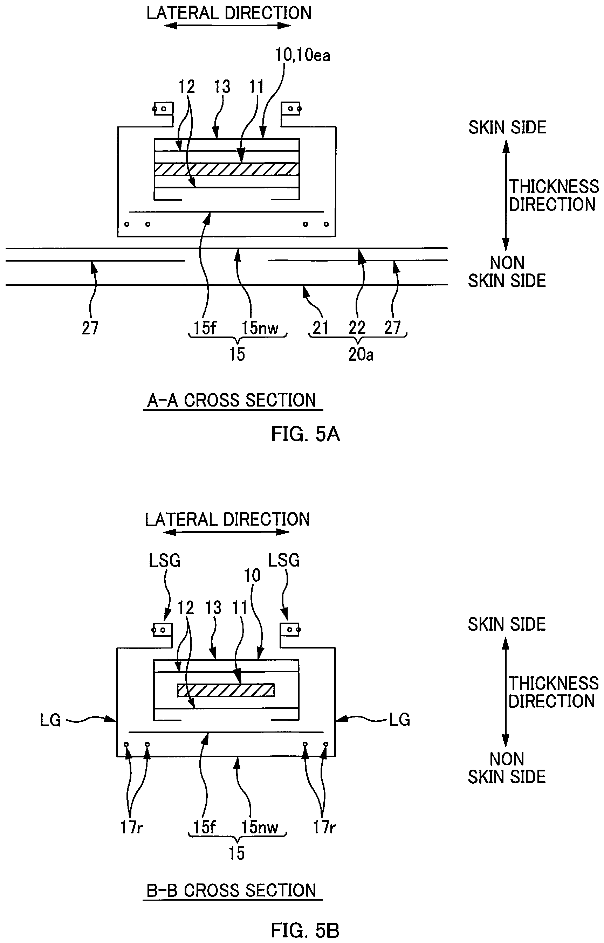

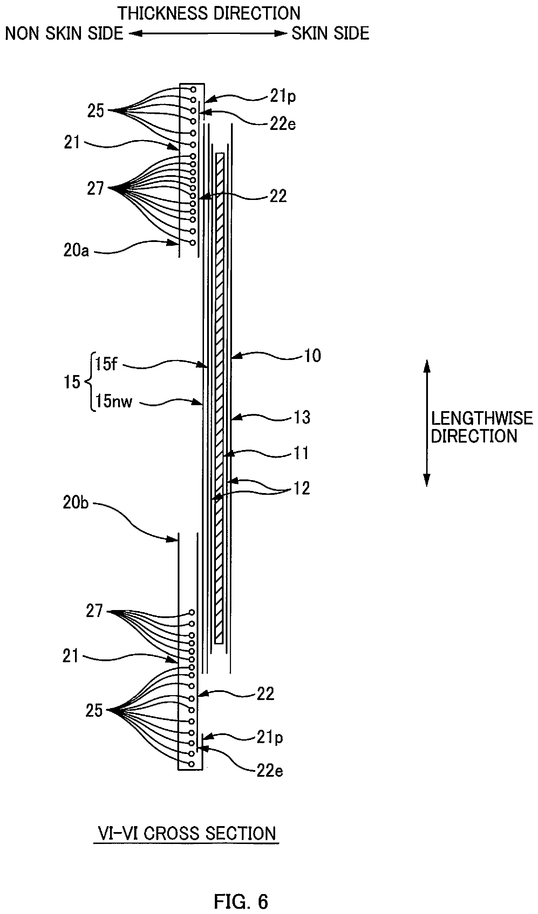

A method of manufacturing a sheet-like member 20as associated with an absorbent article of the present embodiment and a manufacturing apparatus 30 are, for example, is utilized in a manufacturing line of a disposable diaper 1 as an example of the absorbent article. FIG. 2 is a schematic perspective view of a three-piece type disposable diaper 1 as an example of the disposable diaper 1. Further, FIG. 3 is a schematic plan view of the unfolded diaper 1 seen from a skin side, and FIG. 4 is a schematic plan view of the diaper 1 seen from a non-skin side. Further, FIG. 5A is a cross-sectional view taken along line A-A of FIG. 3, FIG. 5B is a cross-sectional view taken along line B-B of FIG. 3, and FIG. 6 is a cross-sectional view taken along line VI-VI of FIG. 3.

As illustrated in FIGS. 3, 5A and 6, this diaper 1 includes a lengthwise direction, a lateral direction and a thickness direction as three directions orthogonal to one another. Further, since the diaper 1 is a so-called three-piece type of diaper, the diaper 1 includes an absorbent main body 10 as a first component which is applied to the crotch portion of the wearer and absorbs excreted materials such as urine, a front band member 20a as a second component which covers the waist of the wearer from the front side, and a back band member 20b as a third component which covers the waist of the wearer from the back side.

In the opened state shown in FIG. 3, the front band member 20a and the back band member 20b are arranged in parallel to each other with a space therebetween, the absorbent main body 10 is extended across both the band members, and end parts 10ea, 10eb of the absorbent main body 10 in the longitudinal direction are joined and fixed to the nearest band members 20a and 20b, respectively, so that the external appearance thereof is in a substantially H shape when seen in a planar view. Further, from this state, the diaper 1 is folded in two at a substantially center part CL10 of the absorbent main body 10 in the longitudinal direction as a folding position, and the band members 20a and 20b that oppose each other in this two-folded state are connected by welding and the like at the part 20ae, 20be (that is, respective end parts 20ae, 20be in the lateral direction) which are parts that are to abut against the wear's flanks, so that these band members 20a and 20b are connected to be in an annular form, thereby making the pull-on diaper 1 in which a waist opening 1HB and a pair of leg openings 1HL, 1HL are formed as shown in FIG. 2.

The absorbent main body 10 includes an absorbent core 11, a liquid-permeable surface sheet 13 which covers the core 11 from the skin side to form a skin-side surface of the absorbent main body 10, and a liquid-impermeable back face sheet 15 which covers the core 11 from the non-skin side to form the non-skin side surface of the absorbent main body 10. The absorbent core 11 is obtained by forming pulp fiber as an example of liquid absorbent fiber and liquid absorbent material such as superabsorbent polymer into a predetermined shape such as a substantially sandglass shape when seen from above. Note that, the core 11 is covered by a liquid-permeable covering sheet 12 in this example, and further, the back face sheet 15 has a two-layer structure including a nonwoven fabric 15nw and a resin film 15f. However, the invention is not limited thereto.

Furthermore, as illustrated in FIG. 3 and FIG. 5B, the absorbent main body 10 may include barrier cuffs LSG, LSG as leak prevention wall portions at respective end parts in the lateral direction for preventing side leakage. Further, stretchable leg cuffs LG, LG may be formed at portions corresponding to the leg opening 1HL, 1HL of the diaper 1 as illustrated in FIG. 2 by disposing elastic members 17r such as elastic strings respectively at both sides of the absorbent main body 10 in the lateral direction.

On the other hand, as illustrated in FIG. 3 and FIG. 4, the front band member 20a and the back band member 20b are each sheet-like members having a substantially rectangular shape when seen from above elongated in the lateral direction, and include an outer layer sheet 21 and an inner layer sheet 22 that are layered in the thickness direction as illustrated in FIG. 5A and FIG. 6. Note that, the inner layer sheet 22 is located on the skin-side in the thickness direction with respect to the outer layer sheet 21. Further, both sheets 21 and 22 are formed by soft sheets such as nonwoven fabric, and in this example, spun-bonded nonwoven fabric is used. However, the invention is not limited thereto. Other types of nonwoven fabric may be used, and further, for example, resin film and woven fabric may be used. Moreover, in this example, although single fiber made of polypropylene (PP) is used as constituent fiber of the nonwoven fabric, the invention is not limited thereto, and single fiber made of other thermoplastic resin such as polyethylene (PE) may be used. Further, a composite fiber with a core sheath structure made of PE and PP may be used.

Then, central regions 20ac, 20bc of the respective band members 20a and 20b in the lateral direction are placed on and joined to the non-skin side surface of the respective end parts 10ea, 10eb of the absorbent main body 10 in the lengthwise direction.

In this example, as illustrated in FIG. 3 and FIG. 6, the outer layer sheet 21 has a planar size that protrudes outwardly from the inner layer sheet 22 in the lengthwise direction. A protruded portion 21P of the outer layer sheet 21 is folded back inwardly in the lengthwise direction and covers an end part 22e of the inner layer sheet 22 in the lengthwise direction. However, the invention is not limited thereto.

Further, stretchability is imparted to the band members 20a and 20b in the lateral direction so that such band members 20a and 20b can properly hold the waist of the wearer when the diaper 1 is worn. However, from the viewpoint of suppressing occurrence of winkles at a position of the absorbent core 11 of the absorbent main body 10 and preventing deterioration of its liquid absorption property and leakage prevention property, non-stretchable regions AL with substantially no stretchability are formed at the regions 20ac and 20bc of the band members 20a and 20b, which are particularly placed on the absorbent core 11 of the absorbent main body 10 as illustrated in FIG. 4.

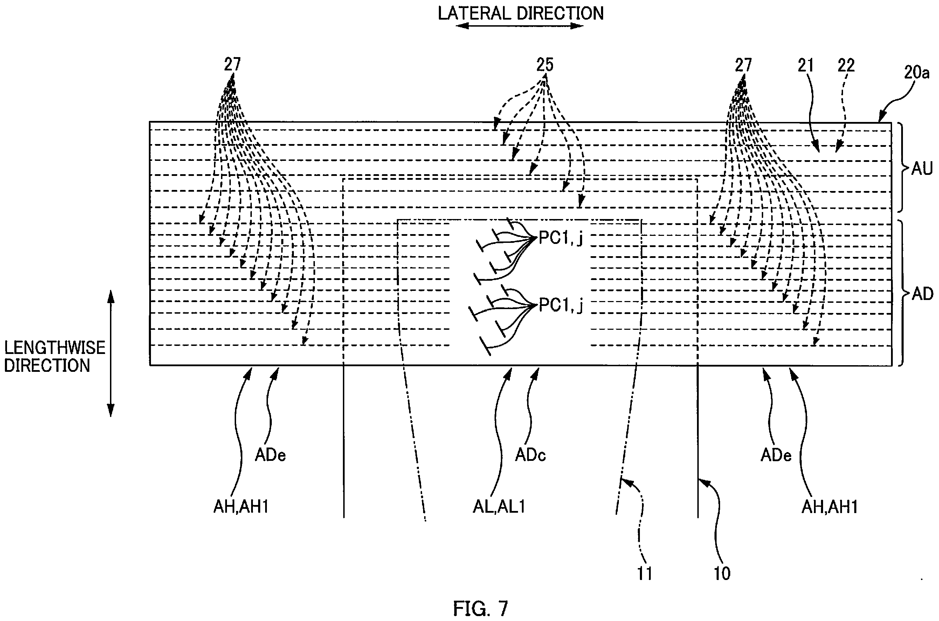

Description of such will be given in the following. Here, the front band member 20a and the back band member 20b have the same basic configuration with each other. Thus, in the description stated below, only the front band member 20a is described, and the description of the back band member 20b is omitted. Further, hereinafter, for convenience of explanation, the front band member 20a is divided into two regions AU and AD in the lengthwise direction as illustrated in the schematic plan view of FIG. 7. That is, the region AU that is located on the waist opening 1HB side in the lengthwise direction is referred to as an "upper region AU", and the region AD that is located on the leg opening 1HB side is referred to as a "lower region AD".

As illustrated in FIG. 7, it can be substantially said that the absorbent core 11 of the absorbent main body 10 only slightly overlaps the upper region AU (corresponding to the second region). Thus, stretchability is imparted to the region AU over the entire length in the lateral direction. Note that, imparting stretchability is realized in such a manner that a plurality of elastic strings 25, 25 ..., which are arranged along the lateral direction in an extended state in the lateral direction, is arranged in the lengthwise direction and fixed to the region AU. In addition, the fixation of the elastic strings 25 to the region AU is performed by hot-melt adhesive applied to the elastic strings 25. In some cases, the fixation may be made by applying the hot-melt adhesive to the region AU. Incidentally, fineness of the elastic strings 25 can be exemplified, for example, by 400 dtex to 1000 dtex, and also the elastic strings 25 can be specifically exemplified by LYCRA (trademark) and the like. Note that, this also applies to elastic strings 27 which are provided in the lower region AD to be described later.

Meanwhile, since most parts of the absorbent core 11 of the absorbent main body 10 are placed on a central region ADc of the lower region AD in the lateral direction, non-stretchable region AL (corresponding to the low stretchable region) with substantially no stretchability in the lateral direction is formed in the central region ADc. Further, the absorbent core 11 only slightly overlaps respective end side regions ADe and ADe located on both sides of the central region ADc, and thus stretchable regions AH, AH (corresponding to the high stretchable region) having higher stretchability than that of the non-stretchable region AL are formed in the end side regions ADe and ADe.

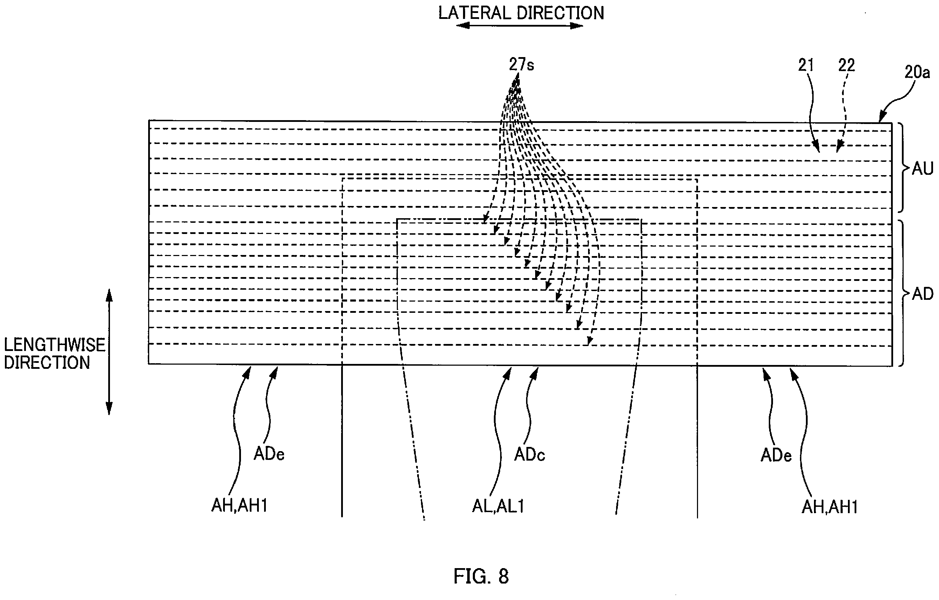

Here, the non-stretchable region AL of the central region ADc and the stretchable regions AH, AH of the respective end side regions ADe and ADe are formed in the following manner. First, as illustrated in FIG. 8, a plurality of elastic strings 27s, 27s ... that is in an extended state in the lateral direction along the lateral direction is arranged in the lengthwise direction between the outer layer sheet 21 and the inner layer sheet 22. Further, the elastic strings 27s are each fixed substantially by hot-melt adhesive in a region AH1 that corresponds to the stretchable region AH in the end side region ADe; however, the elastic strings 27s are each substantially unfixed in a region AL1 that corresponds to the non-stretchable region AL in the central region ADc. Thus, when the band member 20a is pressed from both sides in the thickness direction by a cutter device 50 to be described below in the region AL1 that corresponds to the non-stretchable region AL, so that the respective elastic strings 27s, 27s ... are cut, as illustrated in FIG. 7, the cut elastic strings 27, 27 ... contract to the end side in the lateral direction and substantially remain in the region AH1 that corresponds to the stretchable region AH in the end side region ADe without remaining in the region AL1 that corresponds to the non-stretchable region AL in the central region ADc. In this way, the non-stretchable region AL is formed in the central region ADc and the stretchable region AH is formed in the end side region ADe.

In this example, linear compression-bonding parts j are respectively formed at each position PC1 of the front band member 20a which have been pressed from both sides in the thickness direction when the respective elastic strings 27s, 27s ... are cut. Description of these parts will be given in the following.

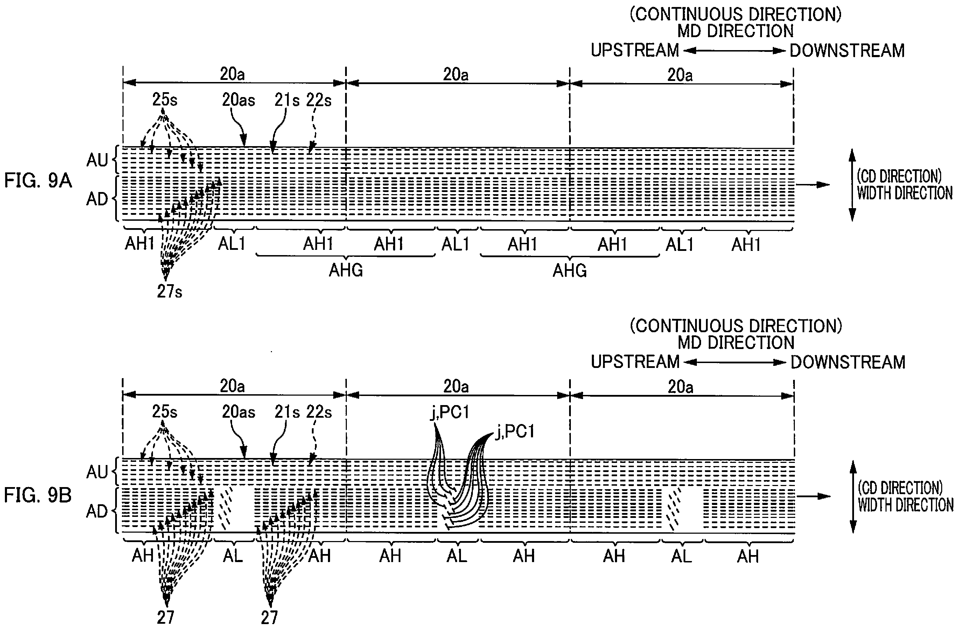

In the manufacturing apparatus 30 of the sheet-like member 20as of the present embodiment, a continuous body 20as made by allowing a plurality of front band members 20a described above to be connected in the lateral direction is formed as an example of the sheet-like member 20as. FIG. 9A is a schematic plan view for explaining the sheet-like member 20as immediately before being transported to the manufacturing apparatus 30. At this point, the sheet-like member 20as is in a state in which a plurality of front band members 20a, 20a ... with the uncut elastic strings 25s, 27s is connected in the lateral direction. That is, the sheet-like member 20as is in a state in which a plurality of elastic string continuous bodies 25s, 25s ... and a plurality of elastic string continuous bodies 27s, 27s ... are interposed between a continuous sheet 21s of the outer layer sheet and a continuous sheet 22s of the inner layer sheet, and the both continuous sheets 21s, 22s are joined. Further, the plurality of elastic string continuous bodies 25s, 25s ... and the plurality of elastic string continuous bodies 27s, 27s ... are arranged in the sheet-like member 20as in the width direction while being in an extended state in a direction in which the sheet-like member 20as is continuous and being along this continuous direction, respectively. Then, one side region of the sheet-like member 20as in the width direction corresponds to the upper region AU of the front band member 20a, and the other side region corresponds to the lower region AD of the front band member 20a. For this reason, in the former one side region AU, the corresponding plurality of elastic string continuous bodies 25s, 25s ... (corresponding to the second elastic member) is fixed by hot-melt adhesive to the respective continuous sheets 21s, 22s associated with the sheet-like member 20as over the entire length in the continuous direction to form the stretchable region over the entire length in the continuous direction. On the other hand, in the latter other side region AD, since the stretchable region AH and the non-stretchable region AL are formed by being arranged in the continuous direction, the corresponding plurality of elastic string continuous bodies 27s, 27s ... is intermittently fixed by hot-melt adhesive to the respective continuous sheets 21s, 22s associated with the sheet-like member 20as in the continuous direction. That is, the elastic string continuous bodies 27s, 27s ... are fixed by hot-melt adhesive in the region AH1 corresponding to the stretchable region AH in the respective continuous sheets 21s, 22s in FIG. 9A; however, are not fixed in the region AL1 corresponding to the non-stretchable region AL. At this time, as illustrated in FIG. 9A, the region AH1 corresponding to the stretchable region AH and the region AL1 corresponding to the non-stretchable region AL appear in the continuous sheet 21s, 22s by being arranged in the continuous direction with a ratio of two to one. This is because the stretchable regions AH, AH are respectively present at both sides of the non-stretchable region AL in the lateral direction in the single-cut front band member 20a in FIG. 7. However, it is not necessary here to distinguish the regions AH1, AH1 corresponding to the two adjacent stretchable regions AH, AH from each other for explanation in FIG. 9A. Thus, hereinafter, description will be given by combining these two regions AH1, AH1 to be one region AHG. In that case, as illustrated in FIG. 9A, the region AHG corresponding to the stretchable regions AH, AH and the region AL1 corresponding to the non-stretchable region AL appear alternately in the sheet-like member 20as in the continuous direction. Further, the region AL1 corresponding to the non-stretchable region AL appears on the sheet-like member 20as in the continuous direction at a product pitch of the diaper 1. Thus, in this manufacturing apparatus 30, the respective elastic string continuous bodies 27s, 27s ... of the sheet-like member 20as are cut in the continuous direction at the product pitch of the diaper 1, thereby alternately forming the stretchable regions AH, AH and the non-stretchable region AL in the sheet-like member 20as in the continuous direction as illustrated in FIG. 9B. As a result, the continuous bodies 20as of the front band member 20a, 20a ... as shown in FIG. 9B are formed.

In the following description, the continuous direction of the sheet-like member 20as is also referred to as an "MD direction", and the width direction of the sheet-like member 20as is also referred to as a "CD direction". Incidentally, the thickness direction of the sheet-like member 20as, the MD direction which is the above-mentioned continuous direction and the CD direction which is the above-mentioned width direction are orthogonal to one another.

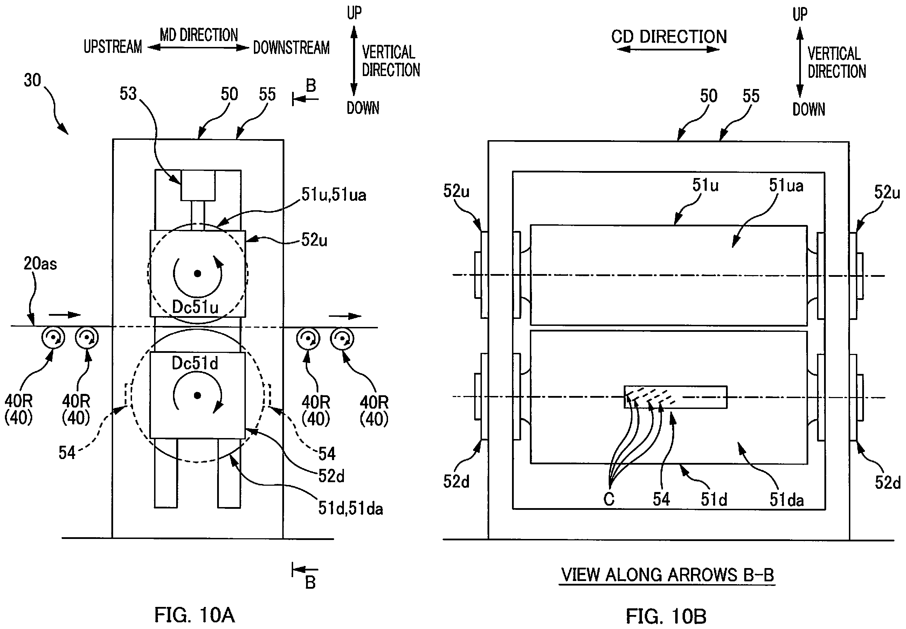

FIG. 10A is a schematic side view of the manufacturing apparatus 30, and FIG. 10B is a view along arrows B-B in FIG. 10A.

As illustrated in FIG. 10A, the manufacturing apparatus 30 includes a transporting device 40 that transports the above-mentioned sheet-like member 20as having the elastic string continuous bodies 27s, 27s ... in the MD direction which serves as the direction of transport; and a cutter device 50 that cuts the above-mentioned elastic string continuous bodies 27s, 27s ... (corresponding to the elastic members) of the above-mentioned sheet-like member 20as.

The transporting device 40 includes, for example, a plurality of transport rollers 40R, 40R .... At least one of the plurality of transport rollers 40R, 40R ... is a drive roller which is driven to rotate about a rotational axis along the CD direction, and also the transport roller 40R other than the drive roller is, for example, a follower roller that corotates by obtaining the rotating force from the sheet-like member 20as. Note that, the transporting device 40 may include a belt conveyor instead of such a transport roller 40R, or in addition to the roller 40R.

The cutter device 50 includes a pair of upper and lower rolls 51u, 51d that rotates about the rotational axis along the CD direction with the respective outer circumferential surfaces 51ua, 51da of both rolls facing each other, and a housing 55 for receiving a reaction force of cutting, which acts on the rolls 51u, 51d when the elastic string continuous bodies 27s, 27s ... are cut.

The pair of upper and lower rolls 51u, 51d is supported by bearing members 52u, 52d provided in the housing 55 respectively, thereby allowing the rolls to rotate about their respective rotational axes which are parallel to the CD direction. Further, the bearing member 52d of the lower roll 51d is immovably fixed in the housing 55, whereas the bearing member 52u of the upper roll 51u is vertically movable in the up-down direction that is a contacting-detaching direction with respect to the lower roll 51d, by an appropriate actuator 53 such as a hydraulic cylinder or an air cylinder, which is fixed to the housing 55. Then, by controlling this actuator 53, it is possible to control the space between the outer circumferential surfaces 51ua, 51da of the pair of upper and lower rolls 51u, 51d and the magnitude of a pressing force (N) in the contacting-detaching direction.

Further, the rolls 51u, 51d are each driven and rotate about the above-mentioned rotational axis by an unshown servomotor as a driving source. That is, the rolls 51u, 51d are driven and rotate in the direction orthogonal to their respective rotational axes, which serves as directions of rotation Dc51u, Dc51d. Thus, the sheet-like member 20as fed between the outer circumferential surfaces 51ua, 51da of the pair of upper and lower rolls 51u, 51d passes between the outer circumferential surfaces 51ua, 51da and is sent out downstream in the MD direction.

Here, in this example, the lower roll 51d is a cutter roll (corresponding to a cutter rotating body) and the upper roll 51u is an anvil roll (corresponding to an anvil rotating body). That is, at least one cutter block 54 is provided on the outer circumferential surface 51da of the lower roll 51d at each angle corresponding to the product pitch of the diaper 1 in the direction of rotation Dc51d. Specifically, in this example, two cutter blocks 54, 54 are provided at each angle of 180° in the direction of rotation Dc51d. Further, a plurality of cutter blades C, C ... is formed to protrude on each of the cutter blocks 54, and also the outer circumferential surface 51ua of the upper roll 51u which is an anvil roll is a smooth surface to receive the above-mentioned cutter blades C, C .... Further, these rolls 51u, 51d are controlled so as to rotate in conjunction with the transport operation of the sheet-like member 20as, so that the cutter roll 51d rotates so as to allow the cutter block 54 to face the region AL1 each time the region AL1 corresponding to the non-stretchable region AL passes by.

Thus, each time the region AL1 corresponding to the non-stretchable region AL of FIG. 9A passes between the outer circumferential surfaces 51ua, 51da of the pair of these rolls 51u, 51d, the region AL1 is pressed between the cutter blades C, C ... of the butter block 54 of the cutter roll 51d and the outer circumferential surface 51ua of the anvil roll 51u. Then, each of the elastic string continuous bodies 27s, 27s ... is cut at each pressed position PC1, and thus the cut elastic strings 27 contract toward the region AH1 corresponding to the high stretchable region AH as illustrated in FIG. 9B. As a result, the non-stretchable region AL and the stretchable region AH are formed in the sheet-like member 20as.

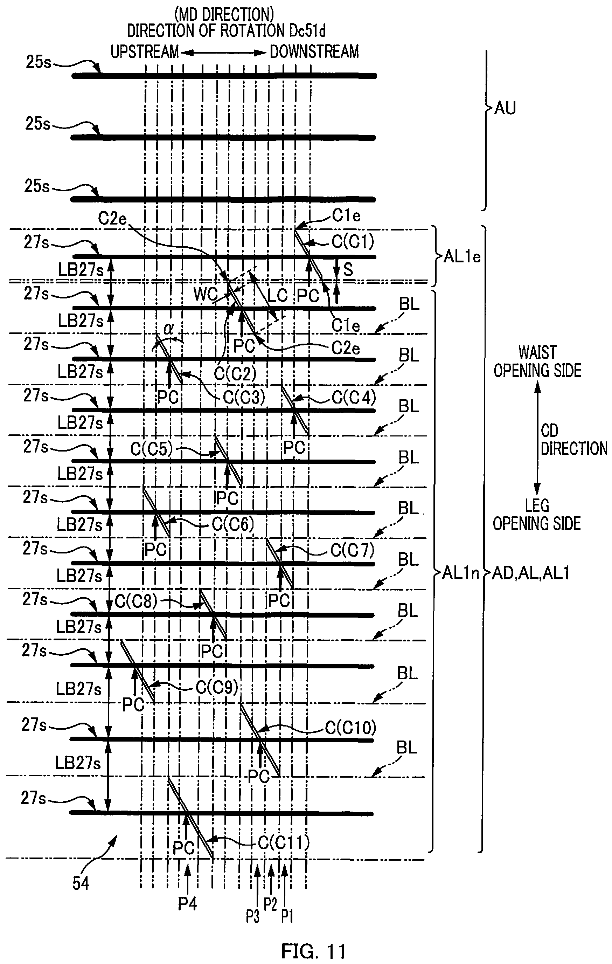

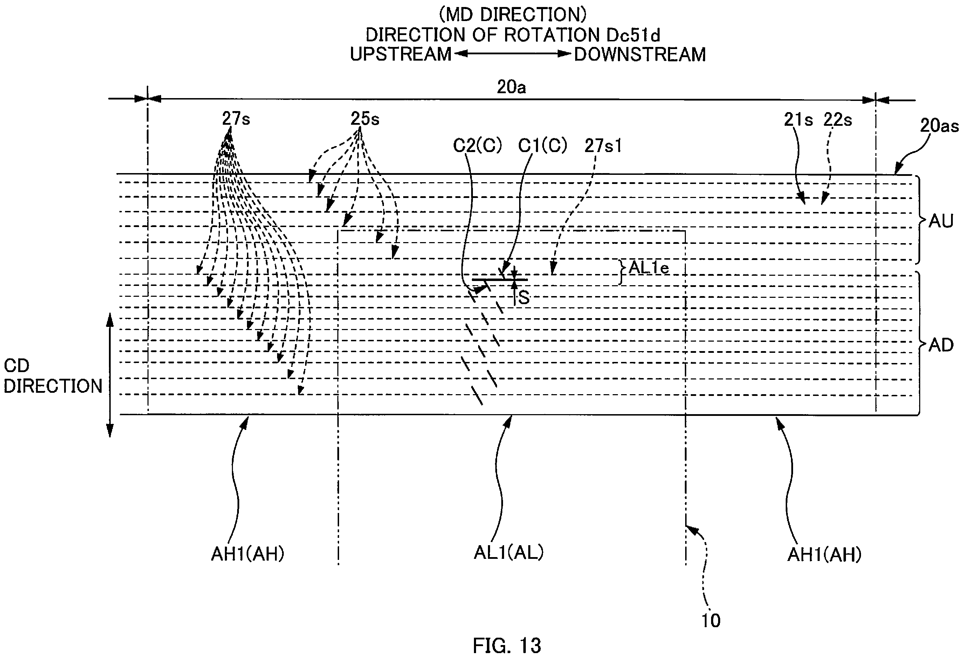

FIG. 11 is an explanatory diagram showing a state of arrangement of a plurality of the cutter blades C, C ... provided in the cutter block 54 of the cutter roll 51d, and is a diagram showing a state in which the outer circumferential surface 51da of the roll 51d is opened on a plane. Note that, in FIG. 11, some of the elastic string continuous bodies 25s, 25s ... of associated with the upper region AU and the elastic string continuous bodies 27s, 27s ... associated with the lower region AD are virtually illustrated together. Further, in FIG. 11, ruled lines parallel to the CD direction are illustrated together by double-dotted chained lines in order to allow the positional relationship of the direction of rotation Dc51d (or the MD direction) to be easily compared. Similarly, ruled lines parallel to the direction of rotation Dc51d (or the MD direction) are illustrated together by double-dotted chained lines in order to allow the positional relationship of the CD direction to be easily compared. This also applies to FIG. 13 to FIG. 16 to be used for explanation later.

Each of the cutter blades C of the cutter block 54 are set apart from the cutter blades C which are adjacent in the CD direction, and also are set apart from the cutter blades C which are adjacent in the direction of rotation Dc51d. The cutting edge of each cutter blade C is linear when seen in a planar view. A length LC of the cutting edge in an extending direction is selected, for example, from the range of 4 mm to 30 mm, and a dimension WC in a blade width direction orthogonal to the extending direction is selected, for example, from the range of 0.1 mm to 1 mm. However, the shape of the cutting edge when seen in a planar view is not limited to the linear shape as stated above. For example, the shape may be a folded-line shape having a zigzag shape, or may be a curved shape such as an arc-line shape. Further, the descriptions such as those relating to the positional relationships of the cutter blade C, for example, arranging continuously or arranging discontinuously in the CD direction the cutter blades C and C which are adjacent in the CD direction, are appropriately used in the following. These descriptions all apply to the positional relationship at the position of the blade tip of the cutter blade C.

Meanwhile, as stated above with reference to FIG. 9A, in the lower region AD of the sheet-like member 20as, eleven elastic string continuous bodies 27s, 27s ... to be cut are arranged along the MD direction and in the CD direction with appropriate intervals between the adjacent elastic string continuous bodies 27s in the CD direction as an example of multiple continuous bodies. Further, in this cutter roll 51d, the rotational operation thereof is performed in conjunction with the transport operation of the sheet-like member 20as, and thus, as illustrated in FIG. 9B, the cutting of these eleven elastic string continuous bodies 27s, 27s ... is performed for each region AL1 corresponding to the non-stretchable region AL in the lower region AD. Also, at that time, each cutting of the elastic string continuous bodies 27s is performed at one position PC in the MD direction as illustrated in FIG. 11.

For this reason, as illustrated in FIG. 11, each one of the cutter blades C is provided for a corresponding one of the elastic string continuous bodies 27s, 27s ... in each of the cutter blocks 54. In this example, since eleven elastic string continuous bodies 27s, 27s ... are arranged in the lower region AD as stated above, each of the cutter blocks 54 is provided with eleven cutter blades C, C .... In this way, each of the cutter blades C cuts a corresponding one elastic string continuous body 27s at one position PC in the MD direction.

At each of the positions PC1 pressed by the respective cutter blades C accompanying the cutting, the continuous sheet 21s that is an outer layer sheet and the continuous sheet 22s of an inner layer sheet associated with the sheet-like member 20as are compressively-bonded, and accordingly, eleven linear compression-bonding parts j, j ..., as an example of multiple compression-bonding parts, are formed so that their respective positions PC1 in the CD direction are shifted to one another as illustrated in FIG. 9B. And, penetration holes are substantially unformed to this position PC1 since the above are compression-bonding parts j. This makes possible to suppress damages that may occur to the sheet-like member 20as caused by cutting the elastic string continuous body 27s. The concept of the above-mentioned linear compression-bonding part j also includes a linear welding part that is formed by welding two continuous sheets 21 and 22 to each other.

Further, as shown in FIG. 11, these eleven cutter blades C provided so that their respective positions in the CD direction shifted to one another, and for convenience of explanation, new reference characters C1 to C11 denoted with respect to each one of the eleven cutter blades C, C ..., in an order in which they are arranged from the one side (the upper side in FIG. 11) to the other side (the lower side in FIG. 11) in the CD direction.

Further, as illustrated in FIG. 11, these eleven cutter blades C are provided so that their respective positions in the CD direction are shifted to one another. And, in FIG. 11, for convenience of explanation, new reference characters C1 to C11 are denoted with respect to each one of the eleven cutter blades C, C ..., in an order in which they are arranged from the one side (the upper side in FIG. 11) to the other side (the lower side in FIG. 11) in the CD direction.

In view of the foregoing conventional problems of the present invention, in the present embodiment, the following positions can be prepared in the region AL1 corresponding to the non-stretchable region AL: a cutting-failure preventing position which can prevent the elastic string continuous bodies 27s from failing to be cut or be cut cleanly (corresponding to the first position); and a shredded-piece preventing position which can prevent producing shredded pieces of the elastic string continuous bodies 27s (corresponding to the second position). It will be described below.

Firstly, a position where a cutting-failure preventing position should be prepared in the region AL1 corresponding to the non-stretchable region AL will be described. In the example of FIG. 11, the upper region AU corresponds to the second region AH2' in which the elastic string continuous bodies 25s, 25s... are not cut (FIG. 1G). The upper region AU is adjacent to the lower region AD. Accordingly, an end part ALle of the region AL1 of the lower region AD, which corresponds to the non-stretchable region AL, is located on the side closer to the upper region AU in the CD direction, and a position of the end part ALle is a position where cutting failure of the elastic string continuous body 27s will not cause a serious problem. Thus, the shredded-piece preventing position is prepared in the end part AL1e. That is, concerning the cutter blade C1 that is to cut the elastic string continuous body 27s located in the end part ALle and the cutter blade C2 that is adjacent to the cutter blade C1 in the CD direction, the cutter blade C1 and the cutter blade C2 are arranged in such a manner that the cutter blades C1 and C2 do not continue in the CD direction. In other words, between these cutter blades C1 and C2, a space S in the CD direction is formed. Specifically speaking, the cutter blades C1 and C2 adjacent in the CD direction respectively have ends C1e, C1e, C2e and C2e in the CD direction, and the space S in the CD direction is formed between the ends C1e and C2e that are closer to each other. Consequently, at the position of the end part AL1e, it is possible to prevent producing shredded pieces of the elastic string continuous body 27s.

The size of the space S in the CD direction is smaller than the minimum of the inter-center distances LB27s, LB27s ... in the CD direction between continuous bodies 27s and 27s adjacent in the CD direction of eleven elastic string continuous bodies 27s that are located in the lower region AD. In this example, the size of the space S in the CD direction is set to 0.1 mm as an example of a value smaller than the minimum. However, the invention is not limited thereto. For example, as long as the size is smaller than the minimum, the size of the space S may be selected from a range from 0.01 mm to 5 mm, or may be selected from a range from 0.05 mm to 3 mm.

There are eleven elastic string continuous bodies 27s provided in the region AL1 corresponding to the non-stretchable region AL, and it is preferable that the size of the space S in the CD direction is smaller than the thickness of the elastic string continuous body 27s positioned nearest the shredded-piece preventing position among the eleven continuous bodies 27s. This makes it possible for two cutter blades C1 and C2 to jointly cut a continuous body 27s even if the continuous body 27s moves to the space S. Thus, at the shredded-piece preventing position, it is possible to effectively prevent cutting failure of the continuous body 27s.

However, the invention is not limited thereto. In some cases, the foregoing relationship may be inverted. That is, the size of the space S in the CD direction is equal to or greater than the thickness of the elastic string continuous body 27s which is positioned nearest the shredded-piece preventing position. This makes it possible to effectively avoid a second cut of the continuous body 27s by these two cutter blades C1 and C2 even if the continuous body 27s moves to the space S. Accordingly, it is possible to more reliably prevent producing shredded pieces of the continuous body 27s. Note that, the foregoing thickness may be measured when the elastic string continuous body 27s does not extend, or may be measured when the elastic string continuous body 27s extends. But, measuring when the elastic string continuous body 27s extends is desirable. The same is applied to the thickness of an elastic string continuous body 27s to be described later.

In this example, as at least one example, three cutter blades C4, C7 and C10 are located in the direction of rotation Dc51d at a position between the cutter blades C1 and C2 which are arranged continuously in the CD direction at the shredded-piece preventing position. Thus, the foregoing two cutter blades C1 and C2 associated with the shredded-piece preventing position are moderately set apart in direction of rotation Dc51d. Accordingly, a moderate amount of time difference can be secured between these two cutter blades C1 and C2: the cutter blade C1, which cuts prior to the cutter blade C2; and the cutter blade C2, which cuts afterward. This makes it possible to effectively prevent producing shredded pieces of the elastic string continuous body 27s, which is caused by cooperation of the cutter blade C1 which cuts prior to the cutter blade C2 and the cutter blade C2 which cuts afterward. That is, after the downstream cutter blade C1 cuts the continuous body 27s, it is possible to prevent the upstream cutter blade C2 from making a second cut to the continuous body 27s being contracting upstream in the direction of rotation Dc51d.

In the example of FIG. 11, in the region AL1 corresponding to the non-stretchable region AL, only the cutter blades C1 and C2 nearest the waist opening 1HB are arranged in such a manner that the cutter blades do not continue in the CD direction. However, the invention is not limited thereto. For example, the damage to non-stretchability of the region AL1 is small, it is possible to increase the number of cutter blades C discontinuously arranged on the side closer to the leg openings 1HL. Specifically speaking, the cutter blade C2 and the cutter blade C3 adjacent to the cutter blade C2 in the CD direction on the side closer to the leg opening 1HL may be arranged in such a manner that the cutter blades do not continue in the CD direction.

Next, a position where a cutting-failure preventing position should be prepared in the region AL1 corresponding to the non-stretchable region AL will be described. As shown in FIG. 11, in the region AL1 corresponding to the non-stretchable region AL, a region except for the end part ALle is defined as a region AL1n. The region AL1n is located apart in the CD direction from the upper region AU. Accordingly, if the elastic string continuous body 27s has failed to be cut or be cut cleanly in the region AL1n, a stretchability that is not negligible is applied to the non-stretchable region AL. Consequently, there is a possibility that non-stretchability of the non-stretchable region AL is significantly damaged. Therefore, in this example, the cutting-failure preventing position is prepared in the region AL1n. That is, among the cutter blades C, C ... which is to cut the elastic string continuous bodies 27s, 27s ... located in the region AL1n, a pair of cutter blades C and C adjacent in the CD direction are arranged continuously in the CD direction. Specifically speaking, in FIG. 11, regarding the cutter blades C2 and C3, the cutter blades C3 and C4, the cutter blades C4 and C5, the cutter blades C5 and C6, the cutter blades C6 and C7, the cutter blades C7 and C8, the cutter blades C8 and C9, the cutter blades C9 and C10, the cutter blades C10 and C11, each pair of the cutter blades are arranged continuously in the CD direction. This makes it possible to effectively prevent all elastic string continuous bodies 27s, 27s ... located in the region AL1n from failing to be cut or be cut cleanly.

In the example of FIG. 11, the cutter blades C and C that are lined continuously in the CD direction are flush with each other at their own boundary positions BL and continue in the CD direction. That is, these cutter blades C and C do not overlap in the CD direction. However, the invention is not limited thereto. That is, these cutter blades C and C may overlap in the CD direction. This makes it possible to more reliably prevent the elastic string continuous body 27s from failing to be cut or be cut cleanly. And, the size of the overlapping in the CD direction may be smaller than the thickness of the elastic string continuous body 27s. This makes it possible to effectively suppress producing shredded pieces.

In FIG. 11, when focusing on the cutter blades C2 and C3 arranged continuously in the CD direction at the cutting-failure preventing position, at least other cutter blades C6, C9 and C11 are located in the direction of rotation Dc51d at a position between these cutter blades C2 and C3. Accordingly, a moderate amount of time difference can be secured between these two cutter blades C2 and C3: the cutter blade C2, which cuts prior to the cutter blade C3; and the cutter blade C3, which cuts afterward. This makes it possible to effectively prevent producing shredded pieces of the elastic string continuous body 27s, which is caused by cooperation of the cutter blade C2 which cuts prior to the cutter blade C3 and the cutter blade C3 which cuts afterward. That is, after the downstream cutter blade C2 cuts the continuous body 27s, it is possible to prevent the upstream cutter blade C3 from making a second cut to the continuous body 27s being contracting upstream in the direction of rotation Dc51d.

The same is applied to each of the following pairs of the cutter blades arranged continuously in the CD direction at their own cutting-failure preventing positions: the cutter blades C3 and C4; the cutter blades C4 and C5; the cutter blades C5 and C6; the cutter blades C6 and C7; the cutter blades C7 and C8; the cutter blades C8 and C9; the cutter blades C9 and C10; and the cutter blades C10 and C11. Accordingly, also in the region AL1n, it is possible to effectively prevent producing shredded pieces of the elastic string continuous body 27s.

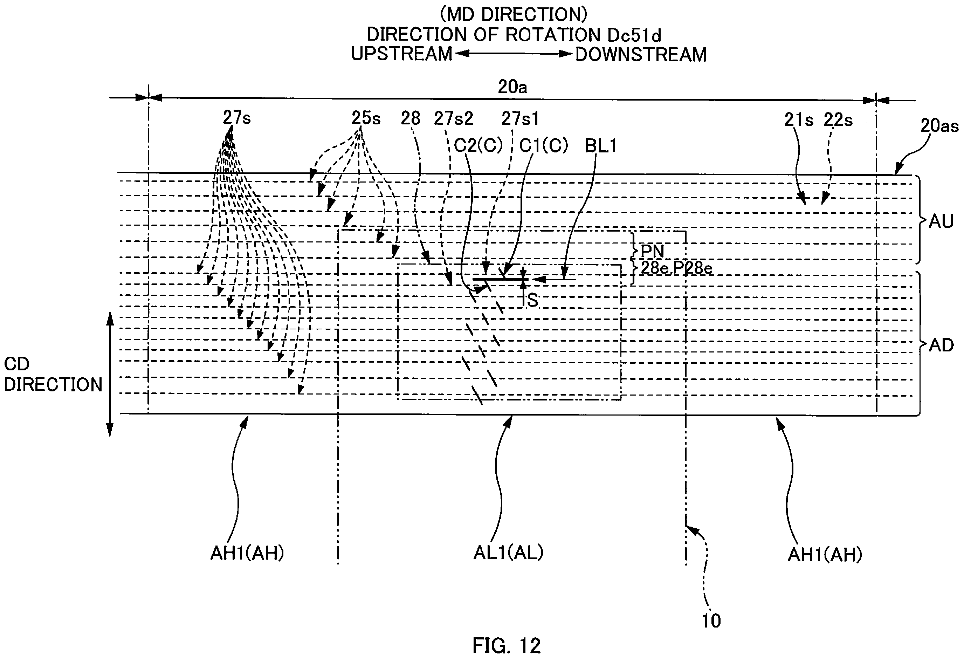

FIG. 12 is a diagram illustrating a first modified example of the shredded-piece preventing position. FIG. 12 shows a schematic plan view of the sheet-like member 20as in which the elastic string continuous bodies 27s, 27s... have not been cut. Also, FIG. 12 virtually shows the cutter blades C, C ... and the absorbent main body 10.

In the first modified example, a single-cut, rectangular illustration sheet 28 (corresponding to the single-cut sheet) is provided for each of the regions AL1 corresponding to the non-stretchable regions AL. With respect thereto, the shredded-piece preventing position is set in an end part 28e of the illustration sheet 28 located on the side closer to the upper region AU in the CD direction. This makes it possible to effectively prevent production of shredded pieces, which is more likely to occur in the end part 28e. The detail thereof is described below.

Firstly, the sheet-like member 20as is likely to deform in the CD direction at a position P28e of the end part 28e of the illustration sheet 28 in the CD direction; this deformation is due to difference of rigidity from a near position PN in which the illustration sheet 28 does not exist. Accordingly, an elastic string continuous body 27s located in the end part 28e is likely to shift in the CD direction. Consequently, there is a possibility that the elastic string continuous body 27sl moves to or near the boundary position BL1 between the cutter blade C1 which is to cut the continuous body 27s1 and the cutter blade C2 adjacent to the cutter blade C1 the CD direction.

In this regard, in the first modified example, the shredded-piece preventing position is set in the end part 28e of the illustration sheet 28. That is, the cutter blade C1 and the cutter blade C2 for the end part 28e are arranged discontinuously in the CD direction. In other words, a space S in the CD direction is disposed between these cutter blades C1 and C2. Accordingly, even when the elastic string continuous body 27s1 shifts in the CD direction and moves to or near the boundary position BL1 of the cutter blades C1 and C2, it is possible to avoid promptly a second cut of the continuous body 27sl by these two cutter blades C1 and C2. This makes it possible to effectively prevent producing shredded pieces of the continuous body 27s.

The illustration sheet 28 is made of a flexible sheet such as resin film. The illustration sheet 28 is basically placed and fixed between the continuous sheet 21s (the outer layer sheet of the sheet-like member 20as) and the continuous sheet 22s (the inner layer sheet of the same). But, in some cases, the illustration sheet 28 may be placed on and fixed to the non-skin side surface of the continuous sheet 21s (the outer layer sheet).

FIG. 13 is a diagram illustrating a second modified example of the shredded-piece preventing position. In the foregoing embodiment, in order to fix the elastic string continuous bodies 25s, 25s ... to the upper region AU, hot-melt adhesive is applied to the elastic string continuous bodies 25s, 25s .... On the other hand, in the second modified, hot-melt adhesive is applied to the upper region AU. Accordingly, difference of rigidity is produced between the upper region AU and the region AL1 corresponding to the non-stretchable region AL, to which adhesive is not applied. The end part ALle of the region AL1 corresponding to the non-stretchable region AL is located on the side closer to the upper region AU in the CD direction, and in the end part AL1e, the sheet-like member 20as is likely to deform in the CD direction due to the foregoing difference of rigidity. Accordingly, the elastic string continuous body 27s1 located in the end part ALle is more likely to shift in the CD direction. Consequently, there is a possibility that the elastic string continuous body 27s1 moves to or near the boundary position BL1 between the cutter blade C1 which is to cut the elastic string continuous body 27s1 and the cutter blade C2 adjacent to the cutter blade C1 in the CD direction.

In this regard, in the second modified example, the shredded-piece preventing position is set in the end part AL1e. That is, the cutter blade C1 and the cutter blade C2 for the end part ALle are arranged discontinuously in the CD direction. In other words, a space S in the CD direction is disposed between these cutter blades C1 and C2. Accordingly, even when the elastic string continuous body 27s1 shifts in the CD direction and moves to or near the boundary position BL1 of the cutter blades C1 and C2, it is possible to avoid promptly a second cut of the continuous body 27s1 by the two cutter blades C1 and C2. This makes it possible to effectively prevent producing shredded pieces of the continuous body 27s1.

When hot-melt adhesive is applied to the upper region AU, the adhesive may be applied in an Ω-shaped pattern or a spiral pattern, or may be applied in a solid manner. However, the invention is not limited thereto. That is, other patterns may be used for applying adhesive.

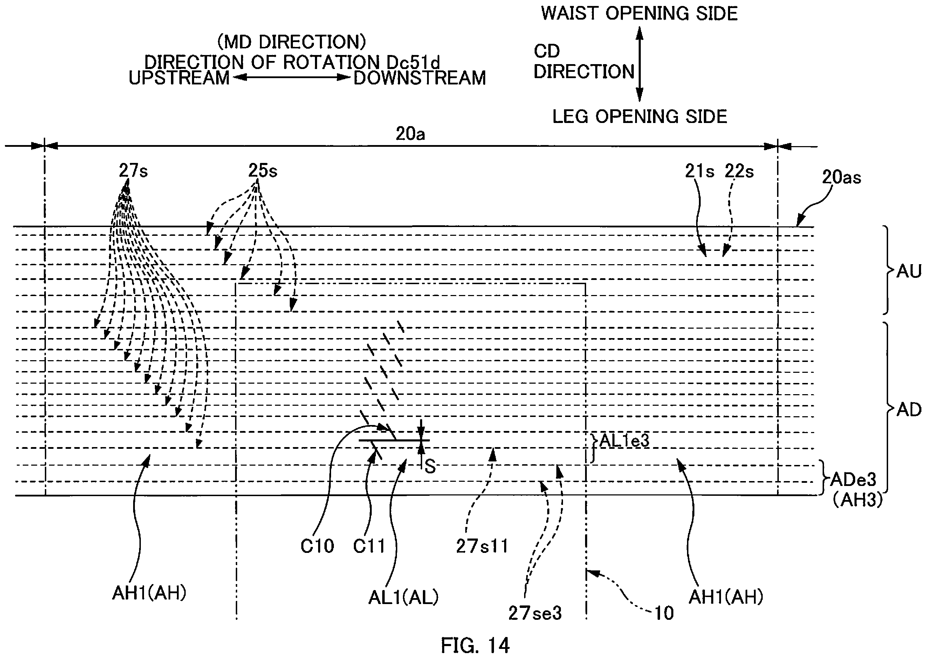

FIG. 14 is a diagram illustrating a third modified example of the shredded-piece preventing position. In the foregoing embodiment, as shown in FIG. 7, in the central region ADc of the lower region AD, non-stretchable region AL is formed throughout substantially the entirety in the CD direction. But, in the third modified example, as shown in FIG. 14, the end part ADe3 of the lower region AD is located on the side closer to the leg opening 1HL in the CD direction, and elastic string continuous bodies 27se3 and 27se3 extending in the MD direction are fixed to the end part ADe3 with hot-melt adhesives, etc. The elastic string continuous bodies 27se3 are not cut and remain being continuous. Thus, similar to the upper region AU, in the end part ADe3, a stretchable region AH3 is formed. In this case, an elastic string continuous body 27s11 is located in the end part AL1e3 of the region AL1 corresponding to the non-stretchable region AL, which is located on the side closer to the leg opening 1HL in the CD direction; and even when the elastic string continuous body 27s11 fails to be cut or be cut cleanly, non-stretchability of the non-stretchable region AL is substantially undamaged for the same reason as mentioned above.

Thus, in the third modified example, the shredded-piece preventing position is set in the end part AL1e3. That is, concerning the cutter blade C11 which is to cut the elastic string continuous body 27s11 located in the end part AL1e3, and the cutter blade C10 adjacent to the cutter blades C11 in the CD direction on the side closer to the waist opening 1HB, the cutter blade C11 and C10 are arranged in such a manner that the cutter blades do not continue in the CD direction. In other words, between these cutter blades C10 and C11, provided is a space S in the CD direction. This makes it possible to prevent producing shredded pieces of the continuous body 27s11.

By the way, in the example of FIG. 11, the arrangement of the cutter blades C is devised so as to reduce the vibration of the cutter roll 51d, which is produced during cutting of the elastic string continuous body 27s. This reduction of the vibration will contribute, to no small extent, to prevention of cutting failure of the elastic string continuous body 27s. Accordingly, the following describes the devises made to the arrangement.

First, when focusing two cutter blades C1 and C4 of FIG. 11, it can be seen that these two cutter blades C1 and C4 have a positional relationship as follows. That is, these two cutter blades C1 and C4 are arranged side by side in the direction of rotation Dc51d, and here, the cutter blade C1 located downstream in the direction of rotation Dc51d overlaps the upstream cutter blade C4 in the direction of rotation Dc51d located at least at a position upstream from the position PC where the cutter blade C1 cuts the elastic string continuous body 27s.

Then, in such a positional relationship, after the previous cutter blade C1 cuts the elastic string continuous body 27s and while the cutter blade C1 is still pressed towards the anvil roll 51u, the subsequent cutter blade C4 that cuts another elastic string continuous body 27s next has already been pressed towards the anvil roll 51u.

Thus, the reaction force of cutting that may act on the cutter roll 51d accompanying the cutting can be received not only by the previous cutter blade C1, but also by the subsequent cutter blade C4 after the cutting. Consequently, the vibration of the cutter roll 51d that may be caused accompanying a fluctuation of the reaction force of cutting after the elastic string continuous body 27s is cut can be reduced by a two-point support state. As a result, the vibration of the cutter roll 51d can be effectively reduced.

Further, in this example, as illustrated in FIG. 11, two cutter blades C2 and C3 are located between the previous cutter blade C1 and the subsequent cutter blade C4 in the CD direction as at least one example, and thus the cutter blades C1 and C4 which become in the above-mentioned two-point support state are disposed greatly apart from each other in the CD direction. That is, a supporting span of the two-point support state becomes large. This may effectively contribute to stabilization of the two-point support state, and thus, the reduction of the vibration of the above-mentioned cutter roll 51d can be further certainly achieved.

Further, when at least one cutter blade C2 or C3 is positioned to a position in the CD direction between the two cutter blades C1 and C4 that consecutively cut in time series as mentioned above, it is possible to consecutively cut elastic string continuous bodies 27s, 27s ... located apart from each other in the CD direction. This makes possible to effectively prevent consecutive cutting multiple elastic string continuous bodies 27s, 27s ... on one side in the CD direction. Consequently, during sequential cutting of the elastic string continuous bodies 27s, the transportation of the sheet-like member 20as is effectively prevented from being unsteady like meandering of the sheet-like member 20as in the CD direction.

As is apparent from FIG. 11, in this example, concerning all of cutter blades C, C ... included in the same cutter block 54 other than the cutter blade C9, which cuts the elastic string continuous body 27s last, there is a cutter blade C corresponding to the foregoing other cutter blade C4 for the two-point supporting state. In addition, at least one another cutter blade C is located in the CD direction between the cutter blades C and C that are in the two-point supporting state.

For example in this case, the cutter blade C9 that is positioned most upstream in the direction of rotation Dc51d corresponds to the foregoing cutter blade C9 that cuts the elastic string continuous body 27s last. Here, two cutter blades C4 and C7 of FIG. 11 are focused on as the cutter blade C other than the cutter blade C9. Among these two cutter blades C4 and C7, the cutter blade C4 located on the downstream side in the direction of rotation Dc51d overlaps the upstream cutter blade C7 in the direction of rotation Dc51d located at least at a position upstream in the direction of rotation Dc51d from a position PC where the cutter blade C4 cuts the elastic string continuous body 27s. Therefore, after the previous cutter blade C4 cuts the elastic string continuous body 27s and while the cutter blade C4 is still pressed towards the anvil roll 51u, the subsequent cutter blade C7 that cuts another elastic string continuous body 27s next has already been pressed towards the anvil roll 51u. Thus, the two cutter blades C4 and C7 are able to be in the two-point support state. In the CD direction, between the previous cutter blade C4 and the subsequent cutter blade C7, two cutter blades C5 and C6 are located as an example of at least one cutter blade. Thus, the cutter blades C4 and C7 which become in the above-mentioned two-point supporting state are set apart in the CD direction.

Similarly, the abovementioned arrangement relationship is also applied to a case in which the cutter blades C7, C10 are focused. Similarly, the same also applies to cases in which the cutter blades C10, C2, the cutter blades C2, C5, the cutter blades C5, C8, the cutter blades C8, C11, the cutter blades C11, C3, the cutter blades C3, C6, and the cutter blades C6, C9 are focused, respectively.

Thus, in this example, any cutter blades C, C ... other than the cutter blade C9 that cuts the elastic string continuous body 27s at the end are able to be in the two-point support state having a large supporting span as described above, thereby enabling the vibration of the cutter roll 51d to be effectively reduced.

However, the invention is not limited thereto. In other words, if at least two cutter blades C, C ... belonging to the same cutter block 54 establish the two-point support state having the large supporting span as described above, the effects that reduce vibration of the cutter roll 51d can be properly received.

In FIG. 11, in a case in which the two cutter blades C2, C5 adjacent to each other in the direction of rotation Dc51d are focused, when the cutter blade C2 located on the downstream side in the direction of rotation Dc51d cut the elastic string continuous body 27s, the cutter blade C5 that has been located on the upstream side has also already been pressed towards the anvil roll 51u. That is, the upstream side cutter blade C5 is present so as to cross, in the direction of rotation Dc51d, the position PC at which the downstream side cutter blade C2 cuts the elastic string continuous body 27s. More specifically, a position indicated by an arrow PC that shows a cutting position of the cutter blade C2 in FIG. 11 is located slightly on the upstream side (left side in the figure) in the direction of rotation Dc51d with respect to the longitudinal ruled line (the ruled line along the CD direction) that is a double-dotted chained line placed on the downstream end (the right end in the figure) of the cutter blade C5 in the direction of rotation Dc51d.

Thus, a reaction force of cutting that may act on the cutter roll 51d can be certainly received not only by the downstream side cutter blade C2 but also by the upstream side cutter blade C5 from the time when the cutter blade C2 performs the cutting. In this way, vibration of the cutter roll 51d that may be caused accompanying a fluctuation of the reaction force of cutting can be promptly reduced by the two-point support state of the downstream side cutter blade C2 and the upstream side cutter blade C5.

Incidentally, the pairs of the cutter blades C, C that satisfy the above matter are also present other than the above case. For example, the two-point support state at the moment of cutting described above is realized even in a pair of cutter blades C7, C10, a pair of cutter blades C8, C11, a pair of cutter blades C3, C6 and the like. Consequently, it is possible for the cutter device 50 to reduce the vibration of the cutter roll 51d more effectively.

Furthermore, in the example of FIG. 11, each direction of the cutter blades C, C is tilted by a tilt angle α having an appropriate magnitude other than 90° from the CD direction for the purpose of forming an overlapping relationship in the direction of rotation Dc51d as described above without any problem, and also not only the magnitude of the tilt angle α but also its direction of tilting are made uniform in all of the cutter blades C, C .... Specifically, in this example, the magnitude of the tilt angle α is 30°, and the direction of tilting is set so that all of the cutter blades C, C ... are each mutually displaced to the same side in the CD direction toward the downstream side in the direction of rotation Dc51d.

When the cutter blades C, C ... are set in this way, change of a separation distance in the CD direction between the cutter blades C, C ... that are to be in the two-point support state can be reduced along the direction of rotation Dc51d, and thus the two-point support state is stabilized.

Further, if the cutter blade C is tilted with respect to the CD direction (corresponding to the direction along an axis of rotation) as stated above, it is possible to ensure a long cutting time for cutting each of the elastic string continuous bodies 27s by the tilted amount of the cutter blade C in comparison with a case in which the cutter blade C is parallel to the CD direction. In this way, it is possible to certainly cut the elastic string continuous bodies 27s.

However, the invention is not limited thereto as long as the cutter blades C, C ... are allowed to be overlapped in the direction of rotation Dc51d. That is, the magnitude of the tilt angle α is not limited to the above-mentioned 30° at all, and the magnitude of the tilt angle α may not be made uniform among all of the cutter blades C, C .... Further, the direction of tilting may not be made uniform among all of the cutter blades C, C ....

Further, in the example of FIG. 11, among all of the cutter blades C1 to C11 belonging to the cutter blocks 54 of the cutter roll 51d, the number of cutter blades C that are simultaneously pressed towards the anvil roll 51u is two or fewer. That is, no more than two cutter blades C are simultaneously pressed. Thus, it is possible to effectively prevent the pressing force applied to the cutter blades C from being distributed to the multiple cutter blades C and reduced, thereby enabling each of the elastic string continuous bodies 27s to be certainly cut.

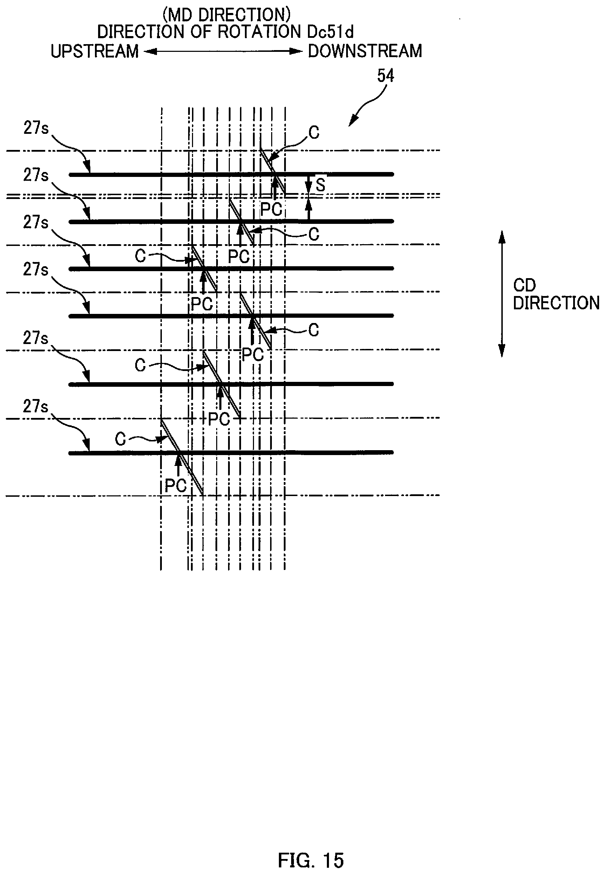

FIG. 15 is a schematic plan view of a first modified example of the arrangement pattern of the cutter blades C, C .... The arrangement pattern of the first modified example is, for example, used for cutting all of six elastic string continuous bodies 27s, 27s ... arranged in the central region ADc of the lower region AD of the back band member 20b as illustrated in FIG. 4. Then, it is apparent from FIG. 15 that this arrangement pattern also satisfies all of the description stated above, and thus the detailed description thereof is omitted.

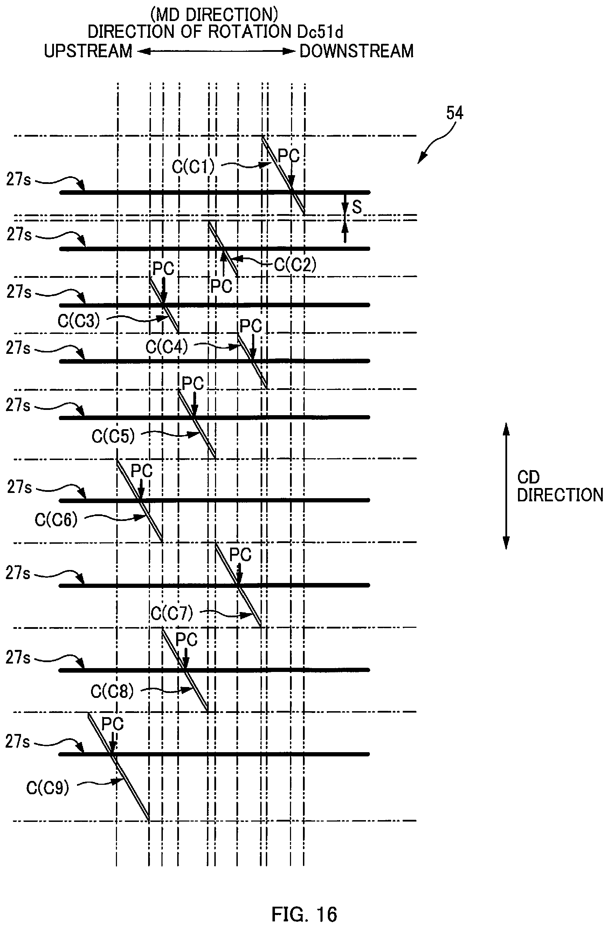

FIG. 16 is a schematic plan view of a second modified example of the arrangement pattern of the cutter blades C, C ....

In the foregoing embodiment, as shown in FIG. 11, among the cutter blades of the cutter block 54, concerning the cutter blades except for the cutter blades C1 and C9 that cut respectively elastic string continuous bodies 27s first and last, in other words, concerning the cutter blades C2 to C8, C10 and C11, the number of cutter blades C that are simultaneously pressed towards the anvil roll 51u is not equal to a certain number.

For example, in the FIG. 11, at each of a first checkup position P1 and a second checkup position P2 in the direction of rotation Dc51d, there are two cutter blades C and C and therefore the number of cutter blades C that are simultaneously pressed towards the anvil roll 51u is 2. At each of a third checkup position P3 and a fourth checkup position P4 in FIG. 11, there is only one cutter blade C and therefore the number of cutter blades C that are simultaneously pressed is not 2. Thus, in the arrangement pattern shown in FIG. 11, concerning the cutter blades C2 to C8, C10 and C11 (the cutter blades except for the cutter blades C1 and C9), the number of cutter blades C that are simultaneously pressed towards the anvil roll 51u is not equal to the certain number.

On the other hand, in the second modified example of FIG. 16, among the cutter blades of the cutter block 54, concerning all other cutter blades C2 to C8, C10 and C11 than the cutter blades C1 and C9 that cut respectively elastic string continuous bodies 27s first and last, the number of cutter blades C that are simultaneously pressed is equal to the certain number.

More specifically, in this example, the cutter blades C1 and C9 located most downstream and most upstream in the direction of rotation Dc51d respectively correspond to cutter blades C and C that cut elastic string continuous bodies 27s first and last respectively. Therefore, when focusing on the cutter blades C2 to C8 (except for two cutter blades C1 and C9), concerning these seven cutter blades C2 to C8, the number of cutter blades C that are simultaneously pressed is equal to the certain number, specifically speaking, the number of cutter blades C is equal to 2 as an example of the certain number.

Accordingly, during cutting, it is possible to effectively prevent distribution of the pressing force that is applied to each cutter blade C, to ensure a great pressing force. And, at the same time, it is possible for the magnitude of the pressing force is equal throughout the seven cutter blades C2 to C8. This makes it possible to effectively prevent cutting failure of the elastic string continuous body 27s.

### === Other Embodiments ===

Although embodiments of the present invention have been described above, the above-described embodiments are intended to facilitate the understanding of the present invention, and the present invention is not to be construed as being limited to the embodiments. And it is needless to say that various modifications and improvements can be made to the present invention without departing from the gist thereof, and equivalents thereof fall within the present invention. For example, the following modifications are possible.

In the foregoing embodiment, as shown in FIG. 9B, at the positions PC1 where the sheet-like member 20as is pressed from both sides by the cutter blades C, the linear compression-bonding parts j are formed. However, the invention is not limited thereto. That is, penetration holes extending through in the thickness direction may be formed at the positions PC1 by cutting the continuous sheets 21s and 22s of the sheet-like member 20as at the positions PC1.

In the foregoing embodiment, the continuous sheet 21s (outer layer sheet) and continuous sheet 22s (inner layer sheet) are described as an example of the continuous sheet associated with the sheet-like member 20as. But, the invention is not limited to a two-layer structure. For example, a single layer structure may be employed, and also three or more layer structure may be employed.

In the foregoing embodiment, the elastic string continuous body 27s is described as an example of the elastic member. However, the invention is not limited thereto. For example, strip-like elastic members such as continuous bodies of flat elastic may be employed.

In the foregoing embodiment, as illustrated in FIG. 11, the plurality of elastic string continuous bodies 27s, 27s ... are exemplified as the plurality of elastic members, and each one of the cutter blades C, C ... is provided for a corresponding one of the plurality of elastic string continuous bodies 27s, 27s .... However, the invention is not limited thereto. For example, one cutter blade C may be provided for corresponding two or three elastic string continuous bodies 27s as an example of two or more elastic string continuous bodies. That is, one cutter blade C may be provided so as to span two elastic string continuous bodies 27s, 27s or three elastic string continuous bodies 27s, 27s and 27s.

In the foregoing embodiment, the cutter roll 51d is exemplified as the cutter rotating body, and the anvil roll 51u is exemplified as an anvil rotating body. However, the invention is not limited thereto. For example, endless belts may be used as the cutter rotating body and the anvil rotating body, respectively.

In the foregoing embodiment, the three-piece type disposable diaper 1 is exemplified as an example of the absorbent article. However, the invention is not limited thereto. For example, the elastic string continuous body 27s provided on the sheet-like member to be used for a two-piece type disposable diaper may be cut by the above-mentioned cutter device 50. Here, the two-piece type disposable diaper is a type of diaper that includes an exterior sheet, as a first component, having a two-layer structure in which a front side portion, a crotch portion and a back side portion are included; and the absorbent main body 10, as a second component, that is fixed on the skin side face of the exterior sheet. In that case, the continuous sheet of the above-mentioned exterior sheet corresponds to the above-mentioned sheet-like member, and the elastic string continuous bodies 27s, 27s ... are each interposed between the two layers included in the continuous body of the exterior sheet.

In the above-stated embodiment, the pull-on disposable diaper 1 is exemplified as an example of the absorbent article. However, the invention is not limited thereto. That is, the absorbent article may also be a tape-type disposable diaper. Note that, the tape-type disposable diaper is a type of diaper that includes a front side portion covering the waist part of the wearer from the front side; and a back side portion covering the waist part thereof from the back side, and that uses fastening tapes for connecting the front side portion and the back side portion. Moreover, the absorbent article is not limited to the above-mentioned disposable diaper 1 at all. That is, the manufacturing method and the manufacturing apparatus 30 of the present invention are applicable as long as the absorbent article is one that uses the sheet-like member 20as as stated above as a material. Thus, the concept of this absorbent article also includes urine absorbing pads and sanitary napkins.

### [Reference Signs List]

1: disposable diaper (absorbent article), 1HB: waist opening, 1HL : leg opening,
10: absorbent main body, 10ea: end part, 10eb: end part,
11: absorbent core, 12: covering sheet,
13: surface sheet, 15: back face sheet, 15f: resin film, 15nw: no nwoven fabric,
17r: elastic member,
20a: front band member, 20ac: central region,
20ae: end part, 20be: end part,
20as: sheet-like member,
20b: back band member,
21: outer layer sheet, 21p: portion, 21s: continuous sheet,
22: inner layer sheet, 22e: end part, 22s: continuous sheet,
25: elastic string, 25s: elastic string continuous body (second e lastic member),
27: elastic string, 27s: elastic string continuous body (elastic member, elastic string),
27s1: elastic string continuous body (elastic member),
27se3: elastic string continuous body, 27s11: elastic string continuous body,
28: illustration sheet (single-cut sheet), 28e: end part,
30: manufacturing apparatus,
40: transporting device, 40R: transport roller,
50: cutter device,
51d: cutter roll (cutter rotating body), 51da: outer circumferent ial surface,
51u: anvil roll (anvil rotating body), 51ua: outer circumferential surface,
52d: bearing member, 52u: bearing member,
53: actuator, 54: cutter block, 55: housing,
C: cutter blade, C1: cutter blade, C1N: non-overlapping portion,
C2: cutter blade, C3: cutter blade, C4: cutter blade, C5: cutter blade,
C6: cutter blade, C7: cutter blade, C8: cutter blade, C9: cutter blade,
C10: cutter blade, C11: cutter blade,
C1e: end, C2e: end,
j: linear compression-bonding part (compression-bonding part),
s: gap,
BL: boundary position, BL1: boundary position,
PC: position, PC1: position,
P28e: position, PN: position,
P1: first checkup position, P2: second checkup position, P3: third checkup position, P4: fourth checkup position,
LSG: barrier cuff,
LG: leg cuff,
CL10: substantially center part
AU: upper region (second region),
AD: lower region, ADc: central region, ADe: side region, ADe3: end part,
AH: stretchable region (high stretchable region),
AH1: region, AHG: region, AH3: stretchable region,
AL: non-stretchable region (low stretchable region),
AL1: region, AL1e: end part, AL1n: region, AL1e3: end part,

## Claims

1. A method of manufacturing a sheet-like member associated with an absorbent article,
the sheet-like member having a high stretchable region and a low stretchable region that has a lower stretchability than the high stretchable region in a continuous direction,
the high stretchable region and the low stretchable region being arranged in the continuous direction,
the method comprising:
transporting a continuous sheet along a direction of transport that is the continuous direction,
the continuous sheet including a plurality of elastic members that are continuous along the continuous direction in an extending state in the continuous direction,
the plurality of elastic members being in a state lined in a CD direction that intersects the continuous direction orthogonally,
the plurality of elastic members being fixed at least to a region corresponding to the high stretchable region; and
forming the high stretchable region and the low stretchable region
so as to be arranged in the direction of transport in the continuous sheet
by cutting each of the plurality of elastic members at a position in the direction of transport in a region corresponding to the low stretchable region and
by allowing the plurality of elastic members to contract toward the region corresponding to the high stretchable region,
wherein
when the continuous sheet to which the plurality of elastic members are fixed passes along the direction of transport between a cutter rotating body and an anvil rotating body that rotate with respective outer circumferential surfaces facing each other,
the plurality of elastic members are cut by pressing the continuous sheet by the anvil rotating body and a plurality of cutter blades on the outer circumferential surface of the cutter rotating body,
the continuous sheet includes a first position and a second position,
the first position being a position at which cutter blades adjacent in the CD direction are arranged continuously in the CD direction,
the second position being a position at which cutter blades adjacent in the CD direction are arranged discontinuously in the CD direction,
among the plurality of elastic members, all of adjacent elastic members that are adjacent in the CD direction are arranged being positioned at an inter-center distance of the adjacent elastic members from each other,
a size of a space in the CD direction between the cutter blades arranged discontinuously in the second position is smaller than a minimum of all of the inter-center distances,
the low stretchable region is a region that overlaps an absorbent core of a disposable diaper serving as the absorbent article.

2. A method of manufacturing a sheet-like member associated with an absorbent article according to claim 1, wherein
the continuous sheet includes a second region that is adjacent in the CD direction to a region corresponding to the low stretchable region and that is adjacent in the CD direction to a region corresponding to the high stretchable region,
a plurality of second elastic members are fixed to the second region,
the plurality of second elastic members being continuous along the continuous direction in an extending state in the continuous direction,
the plurality of second elastic members being in a state lined in the CD direction that intersects the continuous direction,
the second region being defined as a second high stretchable region, and
the second position is set in an end part of the region corresponding to the low stretchable region,
the end part being located on a side closer to the second high stretchable region in the CD direction.

3. A method of manufacturing a sheet-like member associated with an absorbent article according to claim 1 or 2, wherein
the continuous sheet includes a second region that is adjacent in the CD direction to a region corresponding to the low stretchable region and that is adjacent in the CD direction to a region corresponding to the high stretchable region,
a single-cut sheet is fixed to the region corresponding to the low stretchable region and is spaced from another single-cut sheet adjacent in the direction of transport, and
the second position is set in an end part of the single-cut sheet,
the end part being located on a side closer to the second region in the CD direction.

4. A method of manufacturing a sheet-like member associated with an absorbent article according to any one of claims 1 to 3, wherein
the continuous sheet includes a second region that is adjacent in the CD direction to a region corresponding to the low stretchable region and that is adjacent in the CD direction to a region corresponding to the high stretchable region,
adhesive is applied to the second region,
a plurality of second elastic members are fixed to the second region with the adhesive,
the plurality of second elastic members being continuous along the continuous direction in an extending state in the continuous direction,
the plurality of second elastic members being in a state lined in the CD direction that intersects the continuous direction,
the second region is defined as a second high stretchable region,
the plurality of elastic members are not fixed with adhesive to the region corresponding to the low stretchable region, and
the second position is set in an end part of the region corresponding to the low stretchable region,
the end part being located on a side closer to the second region in the CD direction.

5. A method of manufacturing a sheet-like member associated with an absorbent article according to any one of claims 1 to 4, wherein
a size of a space in the CD direction between the cutter blades that are arranged discontinuously at the second position is smaller than a thickness of an elastic member positioned nearest the second position among the plurality of elastic members.

6. A method of manufacturing a sheet-like member associated with an absorbent article according to any one of claims 1 to 4, wherein
a size of a space in the CD direction between the cutter blades that are arranged discontinuously at the second position is equal to or greater than a thickness of an elastic member that is positioned nearest the second position among the plurality of elastic members.

7. A method of manufacturing a sheet-like member associated with an absorbent article according to any one of claims 1 to 6, wherein
the cutter blades that are arranged continuously at the first position overlap in the CD direction.

8. A method of manufacturing a sheet-like member associated with an absorbent article according to any one of claims 1 to 7, wherein
the cutter blade is tilted with respect to a direction along an axis of rotation of the cutter rotating body.

9. A method of manufacturing a sheet-like member associated with an absorbent article according to any one of claims 1 to 8, wherein
among the plurality of cutter blades,
at least one another cutter blade is located in a direction of rotation of the cutter rotating body at a position between the cutter blades that are arranged continuously in the CD direction at the first position.

10. A method of manufacturing a sheet-like member associated with an absorbent article according to any one of claims 1 to 9, wherein
among the plurality of cutter blades,
at least one another cutter blade is located in a direction of rotation of the cutter rotating body at a position between the cutter blades that are arranged discontinuously in the CD direction at the second position.

11. A method of manufacturing a sheet-like member associated with an absorbent article according to any one of claims 1 to 10, wherein
after one cutter blade among the plurality of cutter blades has cut a certain elastic member and while the one cutter blade is still being pressed toward the anvil rotating body,
another cutter blade among the plurality of cutter blades that subsequently cuts another elastic member is already being pressed toward the anvil rotating body, and
at least one cutter blade is positioned in the CD direction between the one cutter blade and the other cutter blade.

12. A method of manufacturing a sheet-like member associated with an absorbent article according to claim 11, wherein
a cutter blade that corresponds to the at least one cutter blade is present for all cutter blades of the plurality of cutter blades other than a cutter blade that cuts the elastic member last.

13. A method of manufacturing a sheet-like member associated with an absorbent article according to claim 11 or 12, wherein
when the one cutter blade cuts the certain elastic member, the other cutter blade is already in a state being pressed toward the anvil rotating body.

14. A method of manufacturing a sheet-like member associated with an absorbent article according to any one of claims 1 to 13, wherein
a number of the cutter blade that are simultaneously pressed toward the anvil rotating body among all of the plurality of cutter blades is two or fewer.

15. An apparatus of manufacturing a sheet-like member associated with an absorbent article,
the sheet-like member having a high stretchable region and a low stretchable region that has a lower stretchability than the high stretchable region in a continuous direction,
the high stretchable region and the low stretchable region being arranged in the continuous direction,
the apparatus comprising:
a transporting device that transports a continuous sheet along a direction of transport that is the continuous direction,
the continuous sheet including a plurality of elastic members that are continuous along the continuous direction in an extending state in the continuous direction,
the plurality of elastic members being in a state lined in a CD direction that intersects the continuous direction orthogonally,
the plurality of elastic members being fixed at least to a region corresponding to the high stretchable region; and
a cutter device that forms the high stretchable region and the low stretchable region
so as to be arranged in the direction of transport in the continuous sheet
by cutting each of the plurality of elastic members at a position in the direction of transport in a region corresponding to the low stretchable region and
by allowing the plurality of elastic members to contract toward the region corresponding to the high stretchable region,
the cutter device including a cutter rotating body and an anvil rotating body,
wherein
when the continuous sheet to which the plurality of elastic members are fixed passes along the direction of transport between the cutter rotating body and the anvil rotating body that rotate with respective outer circumferential surfaces facing each other,
the plurality of elastic members are cut by pressing the continuous sheet by the anvil rotating body and a plurality of cutter blades on the outer circumferential surface of the cutter rotating body,
the continuous sheet includes a first position and a second position,
the first position being a position at which cutter blades adjacent in the CD direction are arranged continuously in the CD direction,
the second position being a position at which cutter blades adjacent in the CD direction are arranged discontinuously in the CD direction,
among the plurality of elastic members, all of adjacent elastic members that are adjacent in the CD direction are arranged being positioned at an inter-center distance of the adjacent elastic members from each other,
a size of a space in the CD direction between the cutter blades arranged discontinuously in the second position is smaller than a minimum of all of the inter-center distances,
the low stretchable region is a region that overlaps an absorbent core of a disposable diaper serving as the absorbent article.

## Patentansprüche

1. Ein Herstellungsverfahren eines lagenartigen Elements, das mit einem absorbierenden Artikel verbunden ist,
wobei das lagenartige Element einen hochdehnbaren Bereich und einen niedrigdehnbaren Bereich aufweist, der eine niedirgere Dehnbarkeit als der hochdehnbare Bereich in einer kontinuierlichen Richtung aufweist,
wobei der hochdehnbare Bereich und der niedrigdehnbare Bereich in kontinuierlicher Richtung angeordnet sind,
wobei das Verfahren umfasst:
Transportieren einer kontinuierlichen Lage entlang einer Transportrichtung, die die kontinuierliche Richtung ist,
wobei die kontinuierliche Lage eine Vielzahl von elastischen Elementen umfasst, die entlang der kontinuierlichen Richtung in einem sich erstreckenden Zustand in der kontinuierlichen Richtung kontinuierlich sind,
wobei sich die Vielzahl der elastischen Elemente in einem Zustand befindet, in dem sie in einer CD-Richtung ausgerichtet sind, die die kontinuierliche Richtung orthogonal schneidet,
wobei die Vielzahl der elastischen Elemente mindestens an einem Bereich befestigt ist, der dem hochdehnbaren Bereich entspricht; und
Ausbilden des hochdehnbaren Bereichs und des niedrigdehnbaren Bereichs,
so dass sie in der Transportrichtung in der kontinuierlichen Lage angeordnet sind,
durch Schneiden jedes der Vielzahl von elastischen Elementen an einer Position in der Transportrichtung in einem Bereich, der dem niedrigdehnbaren Bereich entspricht, und
durch Zulassen der Vielzahl von elastischen Elementen, sich in Richtung des Bereichs zusammenzuziehen, der dem hochdehnbaren Bereich entspricht,
wobei
wenn die kontinuierliche Lage, an der die Vielzahl von elastischen Elementen befestigt ist, entlang der Transportrichtung zwischen einem rotierenden Schneidkörper und einem rotierenden Ambosskörper verläuft, die sich mit jeweils gegenüberliegenden äußeren Umfangsoberflächen drehen,
die Vielzahl von elastischen Elementen durch Drücken der kontinuierlichen Lage durch den rotierenden Ambosskörper und eine Vielzahl von Schneidklingen auf der äußeren Umfangsoberfläche des rotierenden Schneidkörpers geschnitten werden,
die kontinuierliche Lage eine erste Position und eine zweite Position umfasst, wobei die erste Position eine Position ist, an der in CD-Richtung angrenzende Schneidklingen kontinuierlich in CD-Richtung angeordnet sind,
wobei die zweite Position eine Position ist, an der in CD-Richtung benachbarte Schneidklingen diskontinuierlich in CD-Richtung angeordnet sind,
unter der Vielzahl von elastischen Elementen, alle angrenzenden elastischen Elemente, die in der CD-Richtung angrenzend sind, in einem Zwischenmittenabstand der angrenzenden elastischen Elemente von einander angeordnet sind,
eine Größe eines Raumes in CD-Richtung zwischen den in der zweiten Position diskontinuierlich angeordneten Schneidklingen kleiner ist als ein Minimum aller Zwischenmittenabstände,
der niedrigdehnbare Bereich ein Bereich ist, der einen absorbierenden Kern einer Einwegwindel, die als absorbierender Artikel fungiert, überlappt.

2. Ein Herstellungsverfahren eines lagenartigen Elements, das mit einem absorbierenden Artikel gemäß Anspruch 1 verbunden ist, wobei
die kontinuierliche Lage einen zweiten Bereich umfasst, der in CD-Richtung an einen Bereich angrenzt, der dem niedrigdehnbaren Bereich entspricht, und der in CD-Richtung an einen Bereich angrenzt, der dem hochdehnbaren Bereich entspricht,
eine Vielzahl von zweiten elastischen Elementen an dem zweiten Bereich befestigt sind,
wobei die Vielzahl von zweiten elastischen Elementen entlang der kontinuierlichen Richtung in einem sich erstreckenden Zustand in der kontinuierlichen Richtung kontinuierlich ist,
wobei sich die Vielzahl der zweiten elastischen Elemente in einem Zustand befindet, der in der CD-Richtung ausgerichtet ist, die die kontinuierliche Richtung schneidet,
wobei der zweite Bereich als ein zweiter hochdehnbarer Bereich definiert ist, und
die zweite Position in einem Endteil des Bereichs gesetzt ist, der dem niedrigdehnbaren Bereich entspricht,
wobei das Endteil auf einer Seite näher zu dem zweiten hochdehnbaren Bereich in CD-Richtung angeordnet ist.

3. Ein Herstellungsverfahren eines lagenartigen Elements, das mit einem absorbierenden Artikel gemäß Anspruch 1 oder 2 verbunden ist, wobei
die kontinuierliche Lage einen zweiten Bereich umfasst, der in CD-Richtung an einen Bereich angrenzt, der dem niedrigdehnbaren Bereich entspricht, und der in CD-Richtung an einen Bereich angrenzt, der dem hochdehnbaren Bereich entspricht,
eine einfach-geschnittene Lage an dem Bereich befestigt ist, der dem niedrigdehnbaren Bereich entspricht, und von einer anderen einfach-geschnittenen Lage benachbart in Transportrichtung beabstandet ist, und
die zweite Position in einem Endteil der einfach-geschnittenen Lage gesetzt ist,
wobei das Endteil auf einer Seite näher zu dem zweiten Bereich in CD-Richtung angeordnet ist.

4. Ein Herstellungsverfahren eines lagenartigen Elements, das mit einem absorbierenden Artikel gemäß einem der Ansprüche 1 bis 3 verbunden ist, wobei
die kontinuierliche Lage einen zweiten Bereich umfasst, der in CD-Richtung an einen Bereich angrenzt, der dem niedrigdehnbaren Bereich entspricht, und der in CD-Richtung an einen Bereich angrenzt, der dem hochdehnbaren Bereich entspricht,
Klebstoff auf den zweiten Bereich aufgetragen wird,
eine Vielzahl von zweiten elastischen Elementen mit dem Klebstoff an dem zweiten Bereich befestigt sind,
wobei die Vielzahl der zweiten elastischen Elemente entlang der kontinuierlichen Richtung in einem sich erstreckenden Zustand in der kontinuierlichen Richtung kontinuierlich ist,
wobei sich die Vielzahl der zweiten elastischen Elemente in einem Zustand befindet, der in der CD-Richtung ausgerichtet ist, die die kontinuierliche Richtung schneidet,
der zweite Bereich als ein zweiter hochdehnbarer Bereich definiert ist,
die Vielzahl der elastischen Elemente nicht mit Klebstoff an dem Bereich befestigt ist, der dem niedrigdehnbaren Bereich entspricht, und
die zweite Position in einem Endteil des Bereichs gesetzt ist, der dem niedrigdehnbaren Bereich entspricht,
wobei das Endteil auf einer Seite näher zu dem zweiten Bereich in CD-Richtung angeordnet ist.

5. Ein Herstellungsverfahren eines lagenartigen Elements, das mit einem absorbierenden Artikel gemäß einem der Ansprüche 1 bis 4 verbunden ist, wobei
eine Größe eines Raumes in der CD-Richtung zwischen den Schneidklingen, die diskontinuierlich an der zweiten Position angeordnet sind, kleiner ist als eine Dicke eines elastischen Elements, das am nächsten zu der zweiten Position unter der Vielzahl von elastischen Elementen angeordnet ist.

6. Ein Herstellungsverfahren eines lagenartigen Elements, das mit einem absorbierenden Artikel gemäß einem der Ansprüche 1 bis 4 verbunden ist, wobei
eine Größe eines Raumes in der CD-Richtung zwischen den Schneidklingen, die diskontinuierlich an der zweiten Position angeordnet sind, gleich oder größer als eine Dicke eines elastischen Elements ist, das am nächsten an der zweiten Position unter der Vielzahl von elastischen Elementen angeordnet ist.

7. Ein Herstellungsverfahren eines lagenartigen Elements, das mit einem absorbierenden Artikel gemäß einem der Ansprüche 1 bis 6 verbunden ist, wobei
die Schneidklingen, die kontinuierlich an der ersten Position angeordnet sind, sich in CD-Richtung überlappen.

8. Ein Herstellungsverfahren eines lagenartigen Elements, das mit einem absorbierenden Artikel gemäß einem der Ansprüche 1 bis 7 verbunden ist, wobei
die Schneidklinge in Bezug auf eine Richtung entlang einer Drehachse des rotierenden Schneidkörpers geneigt ist.

9. Ein Herstellungsverfahren eines lagenartigen Elements, das mit einem absorbierenden Artikel gemäß einem der Ansprüche 1 bis 8 verbunden ist, wobei
unter der Vielzahl von Schneidklingen,
mindestens eine weitere Schneidklinge in einer Drehrichtung des Schneiddrehkörpers an einer Position zwischen den Schneidklingen angeordnet ist, die an der ersten Position kontinuierlich in CD-Richtung angeordnet sind.

10. Ein Herstellungsverfahren eines lagenartigen Elements, das mit einem absorbierenden Artikel gemäß einem der Ansprüche 1 bis 9 verbunden ist, wobei
unter der Vielzahl von Schneidklingen,
mindestens eine weitere Schneidklinge in einer Drehrichtung des Schneiddrehkörpers an einer Position zwischen den Schneidklingen angeordnet ist, die an der zweiten Position diskontinuierlich in CD-Richtung angeordnet sind.

11. Ein Herstellungsverfahren eines lagenartigen Elements, das mit einem absorbierenden Artikel gemäß einem der Ansprüche 1 bis 10 verbunden ist, wobei
nachdem eine Schneidklinge unter der Vielzahl von Schneidklingen ein bestimmtes elastisches Element geschnitten hat und während die eine Schneidklinge noch gegen den rotierenden Ambosskörpers gedrückt wird,
eine weitere Schneidklinge unter der Vielzahl von Schneidklingen, die anschließend ein weiteres elastisches Element schneidet, bereits gegen den rotierenden Ambosskörper gedrückt wird, und
mindestens eine Schneidklinge in CD-Richtung zwischen der einen Schneidklinge und der anderen Schneidklinge angeordnet ist

12. Ein Herstellungsverfahren eines lagenartigen Elements, das mit einem absorbierenden Artikel gemäß Anspruch 11 verbunden ist, wobei
eine Schneidklinge, die der mindestens einen Schneidklinge entspricht, für alle Schneidklingen der Vielzahl von Schneidklingen vorhanden ist, außer einer Schneidklinge, die das elastische Element als letztes schneidet.

13. Ein Herstellungsverfahren eines lagenartigen Elements, das mit einem absorbierenden Artikel gemäß Anspruch 11 oder 12 verbunden ist, wobei
wenn die eine Schneidklinge das bestimmte elastische Element schneidet, die andere Schneidklinge bereits in einem gegen den rotierenden Ambosskörper gedrückten Zustand ist.

14. Ein Herstellungsverfahren eines lagenartigen Elements, das mit einem absorbierenden Artikel gemäß einem der Ansprüche 1 bis 13 verbunden ist, wobei
eine Anzahl der Schneidklingen unter allen der Vielzahl von Schneidklingen, die gleichzeitig gegen den rotierenden Ambosskörper gedrückt werden, zwei oder weniger ist.

15. Eine Herstellungsvorrichtung eines lagenartigen Elements, das mit einem absorbierenden Artikel verbunden ist,
wobei das lagenartige Element einen hochdehnbaren Bereich und einen niedrigdehnbaren Bereich aufweist, der eine niedrigere Dehnbarkeit als der hochdehnbare Bereich in einer kontinuierlichen Richtung aufweist,
wobei der hochdehnbare Bereich und der niedrigdehnbare Bereich in kontinuierlicher Richtung angeordnet sind,
wobei das Verfahren umfasst:
eine Transportvorrichtung, die eine kontinuierliche Lage entlang einer Transportrichtung, die die kontinuierliche Richtung ist, transportiert,
wobei die kontinuierliche Lage eine Vielzahl von elastischen Elementen umfasst, die entlang der kontinuierlichen Richtung in einem sich erstreckenden Zustand in der kontinuierlichen Richtung kontinuierlich sind,
wobei sich die Vielzahl der elastischen Elemente in einem Zustand befindet, in dem sie in einer CD-Richtung ausgerichtet sind, die die kontinuierliche Richtung orthogonal schneidet,
wobei die Vielzahl der elastischen Elemente mindestens an einem Bereich befestigt ist, der dem hochdehnbaren Bereich entspricht; und
eine Schneidevorrichtung, die den hochdehnbaren Bereich und den niedrigdehnbaren Bereich ausbildet,
so dass sie in der Transportrichtung in der kontinuierlichen Lage angeordnet sind,
durch Schneiden jedes der Vielzahl von elastischen Elementen an einer Position in der Transportrichtung in einem Bereich, der dem niedrigdehnbaren Bereich entspricht, und
durch Zulassen der Vielzahl von elastischen Elementen, sich in Richtung des Bereichs zusammenzuziehen, der dem hochdehnbaren Bereich entspricht,
wobei die Schneidevorrichtung einen rotierenden Schneidkörper und einen rotierenden Ambosskörper umfasst,
wobei
wenn die kontinuierliche Lage, an der die Vielzahl von elastischen Elementen befestigt ist, entlang der Transportrichtung zwischen einem rotierenden Schneidkörper und einem rotierenden Ambosskörper verläuft, die sich mit jeweils gegenüberliegenden äußeren Umfangsoberflächen drehen,
die Vielzahl von elastischen Elementen durch Drücken der kontinuierlichen Lage durch den rotierenden Ambosskörper und eine Vielzahl von Schneidklingen auf der äußeren Umfangsoberfläche des rotierenden Schneidkörpers geschnitten werden,
die kontinuierliche Lage eine erste Position und eine zweite Position umfasst, wobei die erste Position eine Position ist, an der in CD-Richtung angrenzende Schneidklingen kontinuierlich in CD-Richtung angeordnet sind,
wobei die zweite Position eine Position ist, an der in CD-Richtung benachbarte Schneidklingen diskontinuierlich in CD-Richtung angeordnet sind,
unter der Vielzahl von elastischen Elementen, alle angrenzenden elastischen Elemente, die in der CD-Richtung angrenzend sind, in einem Zwischenmittenabstand der angrenzenden elastischen Elemente von einander angeordnet sind,
eine Größe eines Raumes in CD-Richtung zwischen den in der zweiten Position diskontinuierlich angeordneten Schneidklingen kleiner ist als ein Minimum aller Zwischenmittenabstände,
der niedrigdehnbare Bereich ein Bereich ist, der einen absorbierenden Kern einer Einwegwindel, die als absorbierender Artikel fungiert, überlappt.

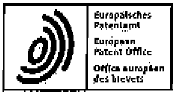

## Revendications

1. Procédé de fabrication d'un élément de type feuille associé à un article absorbant,
l'élément de type feuille présentant une région à haute extensibilité et une région à faible extensibilité qui présente une extensibilité inférieure à la région à haute extensibilité dans un sens continu,
la région à haute extensibilité et la région à faible extensibilité étant agencées dans le sens continu,
le procédé comprenant :
le transport d'une feuille continue le long d'un sens de transport qui correspond au sens continu,
la feuille continue incluant une pluralité d'éléments élastiques qui sont continus le long du sens continu dans un état d'extension dans le sens continu,
la pluralité d'éléments élastiques étant dans un état aligné dans un sens transversal (CD) qui coupe le sens continu orthogonalement,
la pluralité d'éléments élastiques étant fixée au moins à une région correspondant à la région à haute extensibilité ; et
la formation de la région à haute extensibilité et de la région à faible extensibilité de manière à être agencées dans le sens de transport dans la feuille continue en coupant chacun de la pluralité d'éléments élastiques au niveau d'une position dans le sens de transport dans une région correspondant à la région à faible extensibilité et en permettant à la pluralité d'éléments élastiques de se contracter vers la région correspondant à la région à haute extensibilité,
dans lequel
lorsque la feuille continue à laquelle la pluralité d'éléments élastiques est fixée passe le long du sens de transport entre un corps rotatif de coupe et un corps rotatif d'enclume qui tournent avec des surfaces circonférentielles extérieures respectives se faisant face,
la pluralité d'éléments élastiques est coupée par pression de la feuille continue par le corps rotatif d'enclume et une pluralité de lames de coupe sur la surface circonférentielle extérieure du corps rotatif de coupe,
la feuille continue inclut une première position et une seconde position,
la première position étant une position dans laquelle des lames de coupe adjacentes dans le sens transversal (CD) sont agencées de manière continue dans le sens transversal (CD),
la seconde position étant une position dans laquelle des lames de coupe adjacentes dans le sens transversal (CD) sont agencées de manière discontinue dans le sens transversal (CD),
parmi la pluralité d'éléments élastiques, tous les éléments élastiques adjacents qui sont adjacents dans le sens transversal (CD) sont agencés de manière à être positionnés à une distance inter-centre des éléments élastiques adjacents les uns des autres,
une taille d'un espace dans le sens transversal (CD) entre les lames de coupe agencées de manière discontinue dans la seconde position est inférieure à un minimum de toutes les distances inter-centres,
la région à faible extensibilité est une région qui recouvre un noyau absorbant d'une couche jetable servant d'article absorbant.

2. Procédé de fabrication d'un élément de type feuille associé à un article absorbant selon la revendication 1, dans lequel
la feuille continue inclut une seconde région qui est adjacente dans le sens transversal (CD) à une région correspondant à la région à faible extensibilité et qui est adjacente dans le sens transversal (CD) à une région correspondant à la région à haute extensibilité,
une pluralité de seconds éléments élastiques est fixée à la seconde région,
la pluralité de seconds éléments élastiques étant continue le long du sens continu dans un état d'extension dans le sens continu,
la pluralité de seconds éléments élastiques étant dans un état aligné dans le sens transversal (CD) qui coupe le sens continu,
la seconde région étant définie comme une seconde région à haute extensibilité, et
la seconde position est définie dans une partie d'extrémité de la région correspondant à la région à faible extensibilité,
la partie d'extrémité étant située sur un côté plus près de la seconde région à haute extensibilité dans le sens transversal (CD).

3. Procédé de fabrication d'un élément de type feuille associé à un article absorbant selon la revendication 1 ou 2, dans lequel
la feuille continue inclut une seconde région qui est adjacente dans le sens transversal (CD) à une région correspondant à la région à faible extensibilité et qui est adjacente dans le sens transversal (CD) à une région correspondant à la région à haute extensibilité,
une feuille à coupe unique est fixée sur la région correspondant à la région à faible extensibilité et est espacée d'une autre feuille à coupe unique adjacente dans le sens de transport, et
la seconde position est définie dans une partie d'extrémité de la feuille à coupe unique, la partie d'extrémité étant située sur un côté plus près de la seconde région dans le sens transversal (CD).

4. Procédé de fabrication d'un élément de type feuille associé à un article absorbant selon l'une quelconque des revendications 1 à 3, dans lequel
la feuille continue inclut une seconde région qui est adjacente dans le sens transversal (CD) à une région correspondant à la région à faible extensibilité et qui est adjacente dans le sens transversal (CD) à une région correspondant à la région à haute extensibilité,
l'adhésif est appliqué sur la seconde région,
une pluralité de seconds éléments élastiques est fixée à la seconde région avec l'adhésif,
la pluralité de seconds éléments élastiques étant continue le long du sens continu dans un état d'extension dans le sens continu,
la pluralité de seconds éléments élastiques étant dans un état aligné dans le sens transversal (CD) qui coupe le sens continu,
la seconde région est définie comme une seconde région à haute extensibilité,
la pluralité d'éléments élastiques n'est pas fixée avec un adhésif à la région correspondant à la région à faible extensibilité, et
la seconde position est définie dans une partie d'extrémité de la région correspondant à la région à faible extensibilité,
la partie d'extrémité étant située sur un côté plus près de la seconde région dans le sens transversal (CD).

5. Procédé de fabrication d'un élément de type feuille associé à un article absorbant selon l'une quelconque des revendications 1 à 4, dans lequel
une taille d'un espace dans le sens transversal (CD) entre les lames de coupe qui sont agencées de manière discontinue dans la seconde position est inférieure à une épaisseur d'un élément élastique positionné au plus près de la seconde position parmi la pluralité d'éléments élastiques.

6. Procédé de fabrication d'un élément de type feuille associé à un article absorbant selon l'une quelconque des revendications 1 à 4, dans lequel
une taille d'un espace dans le sens transversal (CD) entre les lames de coupe qui sont agencées de manière discontinue dans la seconde position est égale ou supérieure à une épaisseur d'un élément élastique positionné au plus près de la seconde position parmi la pluralité d'éléments élastiques.

7. Procédé de fabrication d'un élément de type feuille associé à un article absorbant selon l'une quelconque des revendications 1 à 6, dans lequel
les lames de coupe qui sont agencées de manière continue dans le sens transversal (CD) dans la première position se chevauchent dans le sens transversal (CD).

8. Procédé de fabrication d'un élément de type feuille associé à un article absorbant selon l'une quelconque des revendications 1 à 7, dans lequel
la lame de coupe est inclinée par rapport à un sens le long d'un axe de rotation du corps rotatif de coupe.

9. Procédé de fabrication d'un élément de type feuille associé à un article absorbant selon l'une quelconque des revendications 1 à 8, dans lequel
parmi la pluralité de lames de coupe,
au moins une autre lame de coupe est située dans un sens de rotation du corps rotatif de coupe dans une position située entre les lames de coupe qui sont agencées de manière continue dans le sens transversal (CD) dans la première position.

10. Procédé de fabrication d'un élément de type feuille associé à un article absorbant selon l'une quelconque des revendications 1 à 9, dans lequel parmi la pluralité de lames de coupe,
au moins une autre lame de coupe est située dans un sens de rotation du corps rotatif de coupe dans une position située entre les lames de coupe qui sont agencées de manière discontinue dans le sens transversal (CD) dans la seconde position.

11. Procédé de fabrication d'un élément de type feuille associé à un article absorbant selon l'une quelconque des revendications 1 à 10, dans lequel après qu'une lame de coupe parmi la pluralité de lames de coupe a coupé un certain élément élastique et pendant que l'une lame de coupe est toujours pressée vers le corps rotatif d'enclume,
une autre lame de coupe parmi la pluralité de lames de coupe qui coupe ensuite un autre élément élastique est déjà pressée vers le corps rotatif d'enclume, et
au moins une lame de coupe est positionnée dans le sens transversal (CD) entre l'une lame de coupe et l'autre lame de coupe.

12. Procédé de fabrication d'un élément de type feuille associé à un article absorbant selon la revendication 11, dans lequel
une lame de coupe qui correspond à l'au moins une lame de coupe est présente pour toutes les lames de coupe de la pluralité de lames de coupe autres qu'une lame de coupe qui coupe l'élément élastique en dernier.

13. Procédé de fabrication d'un élément de type feuille associé à un article absorbant selon la revendication 11 ou 12, dans lequel
lorsque l'une lame de coupe coupe l'élément élastique en question, l'autre lame de coupe est déjà dans un état pressé vers le corps rotatif d'enclume.

14. Procédé de fabrication d'un élément de type feuille associé à un article absorbant selon l'une quelconque des revendications 1 à 13, dans lequel
un nombre de lames de coupe qui sont simultanément pressées vers le corps rotatif d'enclume parmi l'ensemble de la pluralité de lames de coupe est de deux ou moins.

15. Appareil de fabrication d'un élément de type feuille associé à un article absorbant, l'élément de type feuille présentant une région à haute extensibilité et une région à faible extensibilité qui présente une extensibilité inférieure à la région à haute extensibilité dans un sens continu,
la région à haute extensibilité et la région à faible extensibilité étant agencées dans le sens continu,
l'appareil comprenant :
un dispositif de transport qui transporte une feuille continue le long d'un sens de transport qui correspond au sens continu,
la feuille continue incluant une pluralité d'éléments élastiques qui est continue le long du sens continu dans un état d'extension dans le sens continu,
la pluralité d'éléments élastiques étant dans un état aligné dans un sens transversal (CD) qui coupe le sens continu orthogonalement,
la pluralité d'éléments élastiques étant fixée au moins à une région correspondant à la région à haute extensibilité ; et
un dispositif de coupe qui forme la région à haute extensibilité et la région à faible extensibilité de manière à être agencées dans le sens de transport dans la feuille continue
en coupant chacun de la pluralité d'éléments élastiques au niveau d'une position dans le sens de transport dans une région correspondant à la région à faible extensibilité et en permettant à la pluralité d'éléments élastiques de se contracter vers la région correspondant à la région à haute extensibilité,
le dispositif de coupe incluant un corps rotatif de coupe et un corps rotatif d'enclume,
dans lequel
lorsque la feuille continue à laquelle la pluralité d'éléments élastiques est fixée passe le long du sens de transport entre le corps rotatif de coupe et le corps rotatif d'enclume qui tournent avec des surfaces circonférentielles extérieures respectives se faisant face,
la pluralité d'éléments élastiques est coupée par une pression de la feuille continue par le corps rotatif d'enclume et une pluralité de lames de coupe sur la surface circonférentielle extérieure du corps rotatif de coupe,
la feuille continue inclut une première position et une seconde position,
la première position étant une position dans laquelle des lames de coupe adjacentes dans le sens transversal (CD) sont agencées de manière continue dans le sens transversal (CD),
la seconde position étant une position dans laquelle des lames de coupe adjacentes dans le sens transversal (CD) sont agencées de manière discontinue dans le sens transversal (CD),
parmi la pluralité d'éléments élastiques, tous les éléments élastiques adjacents qui sont adjacents dans le sens transversal (CD) sont agencés de manière à être positionnés à une distance inter-centre des éléments élastiques adjacents les uns des autres,
une taille d'un espace dans le sens transversal (CD) entre les lames de coupe agencées de manière discontinue dans la seconde position est inférieure à un minimum de toutes les distances inter-centres,
la région à faible extensibilité est une région qui recouvre un noyau absorbant d'une couche jetable servant d'article absorbant.
